# EUROPEAN PATENT SPECIFICATION

(11) **EP 2 623 097 B1**
(45) Date of publication and mention of the grant of the patent: **15.07.2015**
(21) Application number: 13153655.9
(22) Date of filing: 01.02.2013
(51) Int. Cl.: A61K 9/51

(54) **Solid lipid nanoparticles including elastin-like polypeptides and use thereof**
Feste Lipidnanopartikel mit elastinartigen Polypeptiden und Verwendung davon
Nanoparticules lipidiques solides comprenant des polypeptides semblables à de l'élastine et son utilisation

(30) Priority: 01.02.2012 KR 20120010505; 07.01.2013 KR 20130001792
(43) Date of publication of application: 07.08.2013
(73) Proprietor: Samsung Electronics Co., Ltd, Gyeonggi-do 443-742 (KR)
(72) Inventor: Park, Sun-min, Yongin-si, Gyeonggi-do (KR); Kim, Hyun-ryoung, Yongin-si, Gyeonggi-do (KR); Park, Jae-chan, Yongin-si, Gyeonggi-do (KR); Chae, Su-young, Yongin-si, Gyeonggi-do (KR)
(74) Representative: Grünecker Patent- und Rechtsanwälte PartG mbB

(56) References cited:
- ALURI S. ET AL.: "Elastin-Like Peptide Amphiphiles From Nanofibers with Tunable Length", BIOMACROMOLECULES, vol. Aluri S. et Al., 31 July 2012 (2012-07-31), pages 2645-2654, XP002696594,
- WU, Y. ET AL.: "Fabrication of Elastin-Like Polypeptide Nanoparticles for Drug Delivery by Electrosprayin", BIOMACROMOLECULES, vol. 10, 12 October 2008 (2008-10-12), pages 19-24, XP002696595,
- HERRERO-VANRELL R ET AL: "Self-assembled particles of an elastin-like polymer as vehicles for controlled drug release", JOURNAL OF CONTROLLED RELEASE, ELSEVIER, AMSTERDAM, NL, vol. 102, no. 1, 20 January 2005 (2005-01-20), pages 113-122, XP027664501, ISSN: 0168-3659 [retrieved on 2005-01-20]
- SERGEY E PARAMONOV ET AL: "Modulation of Peptide-Amphiphile Nanofibers via Phospholipid Inclusions", BIOMACROMOLECULES, AMERICAN CHEMICAL SOCIETY; US, vol. 7, no. 1, 9 January 2006 (2006-01-09) , pages 24-26, XP002688929, ISSN: 1525-7797, DOI: 10.1021/BM050798K [retrieved on 2005-12-17]

<110> Samsung Electronics Co., Ltd.
<120> Solid lipid nanoparticle including elastin-like polypeptides and use thereof
<130> EP86366FZTRpau
<140> not yet assigned
   <141> herewith
<150> KR 10-2013-0001792
   <151> 2013-01-07
<150> KR 10-2012-0010505
   <151> 2012-02-01
<160> 9
<170> KopatentIn 2.0
<210> 1
   <211> 5
   <212> PRT
   <213> Artificial Sequence
<220>
   <223> elastin-like polypeptide unit sequence
<220>
   <221> VARIANT
   <222> (4)
   <223> Xaa denotes amino acid other than proline
<400> 1
   Val Pro Gly Xaa Gly
   1 5
<210> 2
   <211> 5
   <212> PRT
   <213> Artificial Sequence
<220>
   <223> elastin-like polypeptide unit sequence
<220>
   <221> VARIANT
   <222> (3)
   <223> Xaa denotes amino acid other than proline
<400> 2
   Pro Gly Xaa Gly Val
   1 5
<210> 3
   <211> 5
   <212> PRT
   <213> Artificial Sequence
<220>
   <223> elastin-like polypeptide unit sequence
<220>
   <221> VARIANT
   <222> (2)
   <223> Xaa denotes amino acid other than proline
<400> 3
   Gly Xaa Gly Val Pro
   1 5
<210> 4
   <211> 5
   <212> PRT
   <213> Artificial Sequence
<220>
   <223> elastin-like polypeptide unit sequence
<220>
   <221> VARIANT
   <222> (1)
   <223> Xaa denotes amino acid other than proline
<400> 4
   Xaa Gly Val Pro Gly
   1 5
<210> 5
   <211> 5
   <212> PRT
   <213> Artificial Sequence
<220>
   <223> elastin-like polypeptide unit sequence
<220>
   <221> VARIANT
   <222> (5)
   <223> Xaa denotes amino acid other than proline
<400> 5
   Gly Val Pro Gly Xaa
   1 5
<210> 6
   <211> 5
   <212> PRT
   <213> Artificial Sequence
<220>
   <223> Elastin-like polypeptide
<400> 6
   Val Pro Gly Val Gly
   1 5
<210> 7
   <211> 5
   <212> PRT
   <213> Artificial Sequence
<220>
   <223> Elastin-like polypeptide
<400> 7
   Val Pro Gly Ala Gly
   1               5
<210> 8
   <211> 5
   <212> PRT
   <213> Artificial Sequence
<220>
   <223> Elastin-like polypeptide unit sequence
<400> 8
   Val Pro Gly Lys Gly
   1               5
<210> 9
   <211> 40
   <212> PRT
   <213> Artificial Sequence
<220>
   <223> Elastin-like polypeptide unit sequence; the nitrogens at the N-terminal end and lysine side chains are conjugated to stearoyl groups and C-terminal end is modified to -NH2
<400> 9
   Val Pro Gly Lys Gly Val Pro Gly Val Gly Val Pro Gly Val Gly Val
   1               5                   10                  15
   Pro Gly Val Gly Val Pro Gly Lys Gly Val Pro Gly Val Gly Val Pro
               20                  25                  30
   Gly Val Gly Val Pro Gly Val Gly
           35                  40

## Description

### BACKGROUND

### 1. Field

The present disclosure relates to solid lipid nanoparticles (SLNs) including elastin-like polypeptides and a use thereof.

### 2. Description of the Related Art

Solid lipid nanoparticles (SLNs) were developed as an alternative carrier system to emulsions, liposomes, and polymeric nanoparticles. SLNs can provide advantages including stabilization of incorporated compounds, controlled release, occlusivity, and film formation on skin, including in vivo effects on the skin. Also, when a biological lipid is used, SLNs provide excellent biocompatibility and biodegradability and high storage stability.

SLNs are conventionally prepared by a melting/solidification process, wherein the lipid is first melted, dispersed in water and then cooled to solidify the lipid particles. Alternatively, SLNs are conventionally produced using an emulsion process akin to the formation of polymeric microparticles, wherein the lipids are dissolved in a solvent, emulsified, and then dispersed in an aqueous solution containing an emulsifying agent to harden the SLNs. The role of the emulsifying agent is to stabilize the SLNs.

However, since SLNs are overly stable, releasing of a drug may take a prolonged period of time. Therefore, in order to overcome this deficiency, a method that provides effective drug release is in demand.

Wu, Y. et al., "Fabrication of Elastin-like Polypeptide Nanoparticles for Drug Delivery by Electrospraying", Biomacromolecules 2009, vol. 10, pages 19-24, published October 12, 2008, discloses elastin-like polypeptide nanoparticles produced by mixing a solution of elastin-like polypeptides with a Doxorubicin solution, followed by electrospraying the resulting mixture.

### SUMMARY

Provided are solid lipid nanoparticles (SLNs) including elastin-like polypeptides (ELPs).

Provided is a pharmaceutical composition including SLNs including ELPs.

Provided is a SLN containing an agent for use in delivering the agent to a target site in a subject.

### BRIEF DESCRIPTION OF THE DRAWINGS

These and/or other aspects will become apparent and more readily appreciated from the following description of the embodiments, taken in conjunction with the accompanying drawings of which:
FIG. 1 illustrates a sparingly water-soluble substance release profile of a solid lipid nanoparticle (SLN) including methyl red (MR), which is a sparingly water-soluble substance, that is prepared in the same manner as Example 1;
FIG. 2 illustrates size of the SLN according to an amount of the surfactant prepared in the same manner as Example 2;
FIG. 3 illustrates transmission electron microscopy (TEM) images of a SLN not including coumarin-6 (A) and a SLN including coumarin-6 (B);
FIG. 4 illustrates a SLN size according to a type of lipids and an amount of a surfactant;
FIG. 5 illustrates a temperature sensitivity of particle dispersion according to whether cholesteryl oleate, which serves as a stabilizing agent of the SLN, is inserted in a particle core or not in the same manner as Example 7;
FIG. 6 shows a temperature sensitivity of drug release of the SLN including an elastin-like protein (ELP), in which Paclitaxel is incorporated as described in Example 8; and
FIG. 7 illustrates the results of in-cell delivery of coumarine-6 by using the SLN in the same manner as Example 9.

### DETAILED DESCRIPTION

Reference will now be made in detail to embodiments, examples of which are illustrated in the accompanying drawings, wherein like reference numerals refer to like elements throughout. In this regard, the present embodiments may have different forms Accordingly, the embodiments are merely described below, by referring to the figures, to explain aspects of the present description. As used herein, the term "and/or" includes any and all combinations of one or more of the associated listed items.

According to an aspect of the present invention, a solid lipid nanoparticle (SLN) includes an elastin-like polypeptide (ELP) conjugated to a hydrophobic moiety, and a lipid.

As used herein the term "elastin-like polypeptide" refers to a class of amino acid polymers that undergo a conformation change dependent upon temperature. In an embodiment of the present invention, the ELP may be polymers exhibiting an inverse phase transitioning behavior. An inverse phase transitioning behavior indicates that an ELP is soluble in aqueous solutions below an inverse transition temperature (Tₜ), but the ELP is insoluble as a temperature is raised higher than Tₜ. As the temperature increases, an ELP may be transformed from elongated chains that are highly soluble to tightly folded aggregates with greatly reduced solubility. Such an inverse phase transition may be induced as an ELP structure has more β-turn structures and distorted β-structures when the temperature increases. For example, phase transition of the ELP may occur at a temperature within a range from about 10°C to about 70°C, or from about 39 °C to about 70 °C.

In the SLN, an inverse phase transitioning behavior may destroy the SLN due to shrinkage and self-assembly of the ELP as the temperature rises from a temperature lower than Tₜ of ELP to a higher temperature. Destroying the SLN may increase release of an active agent included in the SLN. Thus, the active agent included in the SLN may be released from the SLN to outside with a higher sensitivity to temperature. However, one or more embodiments of the present invention are not limited to any particular mechanism.

Destruction of a SLN due to inverse phase transitioning behavior of an ELP may differ depending on a phase transition temperature of a lipid constituting the SLN and an ELP. A lipid exists in a gel phase below the phase transition temperature and in a liquid (crystalline) phase above the phase transition temperature. When a lipid exists in a gel phase, destruction of a SLN may not occur or may be limited, although a structure of ELP changes to have β-turn structures due to the inverse phase transitioning behavior. On the other hand, when a lipid exists in a liquid phase, destruction of a SLN may be induced as a structure of ELP changes to have β-turn structures due to an inverse phase transitioning behavior. That is, when a lipid exists in a liquid phase rather than in a gel phase, inverse phase transition induces destruction of a SLN more efficiently. Therefore, a releasing temperature of an active agent contained in a SLN may be controlled by adjusting a phase transition temperature of a lipid and an inverse phase transition temperature of an ELP. For example, a phase transition temperature of an ELP, a lipid constituting a SLN, and a SLN may each be within a range from about 10 °C to about 70 °C, for example, from about 35 °C to about 70 °C, from about 39 °C to about 45 °C, or from about 39 °C to about 60 °C.

The ELP conjugated to a hydrophobic moiety may be conjugated to an N-terminus or C-terminus of an amino acid sequence, a side chain of an N-terminus amino acid residue, a side chain of a C-terminus amino acid residue, or a side chain of an amino acid residue between the N- and C-terminals. For example, the hydrophobic moiety is conjugated to an N-terminus or a C-terminus, and at least one hydrophobic moiety may be conjugated per one ELP molecule. For example, the hydrophobic moiety such as hydrocarbon group, carbonyl containing group such as acyl group and alkoxy group may be conjugated to the ELP by an amine bond or an amide bond with the nitrogen atom at the N-terminus or by an ester bond with the carbonyl group at the C-terminus of the ELP. Here, the hydrophobic moiety may be a hydrocarbon group or a carbonyl containing group such as acyl group or alkoxy group having 4 to 30 carbon atoms, for example, 14 to 24 carbon atoms or 16 to 24 carbon atoms.

An ELP may be defined by its amino acid sequence. For example, a part of or an entire ELP may include one or more repeating units which may be one selected from VPGXG (SEQ ID NO: 1), PGXGV (SEQ ID NO: 2), GXGVP (SEQ ID NO: 3), XGVPG (SEQ ID NO: 4), GVPGX (SEQ ID NO: 5) and a combination thereof, where V is valine, P is proline, G is glycine, and X is any natural or non-natural amino acid except proline. Here, X in each repeating unit may be the same or a different amino acid. The repeating units may be separated by one or more amino acids that do not remove a phase transition property of an obtained ELP in the ELP, or an end portion of the ELP may include the one or more amino acids , for example other than the repeating units. A molar ratio of the repeating units verses the other amino acids may be about 0.1 to about 99.9% of the repeating units out of both the repeating units and the other amino acids. The selected repeating unit may be repeated twice or more, for example, 2 to 200 times.

In an embodiment of the present invention, the ELP may be blocks where VPGXG, PGXGV, GXGVP, XGVPG, GVPGX or a combination thereof is tandemly repeated, or the ELP may include blocks where VPGXG, PGXGV, GXGVP, XGVPG, GVPGX or combinations thereof is tandemly repeated. As long as the inverse phase transition behavior is maintained, the ELP may comprise VPGXG, PGXGV, GXGVP, XGVPG, GVPGX or combinations thereof and may include another portion in a molecule, for example,one or more amino acids such alanine and glycine between the repeating units, and/or at either end or both ends of the ELP. An N-terminus or C-terminus of the ELP may be linked with a hydrophobic moiety. Also, a hydrophobic moiety may be conjugated to an ELP by linking with a reactive group among a side chain of amino acid residue in the ELP. The reactive group may be an amino group, a hydroxyl group, a thiol group, or a carboxyl group. The other terminus not linked with a hydrophobic moiety may be blocked or unblocked. For example, when a hydrophobic moiety and an ELP are linked via the N-terminus of the ELP, a carboxyl group of the C-terminus of ELP may be blocked or unblocked. The blocking may be enabled by linking or interacting with a material that may be biocompatible, non-immunogenic, helpful in a specific delivery, or escapable from a biological degradation system. For example, the blocking may be enabled by an amide bond formed by binding a carboxyl group of a C-terminus of ELP and an amino group. The amino group may be derived from an ammonia molecule, a primary amine, a secondary amine, or a tertiary amine. The primary, secondary, or tertiary amine may each have 1 to 18 carbon atoms, for example, 1 to 6 carbon atoms. X may be valine or alanine.

The repeating units may be each independently included in an ELP with one or more integer number of repetitions. The number of repetitions may be each independently an integer of 2 to 200, 2 to 100, 2 to 80, 2 to 60, 2 to 40, 2 to 10, 2 to 12, 2 to 8, 2 to 6, 4 to 100, 8 to 80, 10 to 60, 12 to 40, 20 to 40, 4 to 10, 4 to 8, or 4 to 6.

The hydrophobic moiety may be a molecule having a property of immobilizing the ELP to the lipid by interacting with lipid molecules. The interaction may be covalent or non-covalent bonding. The interaction may be a hydrophobic interaction, a Van der Waals interaction, ionic bonding, or hydrogen bonding. The hydrophobic moiety may be partially or entirely identical to the lipid or may be another lipid.

The hydrophobic moiety may include a molecule only containing a hydrophobic region or an amphipathic molecule containing both hydrophilic and hydrophobic regions. In the amphipathic molecule, the hydrophobic region may be arranged inwardly of the lipid, and the hydrophilic region may be arranged outwardly of the lipid that enables linking with an ELP. Here, "outwardly" indicates a direction away from a center of a SLN. The moiety may be packed, embedded, or dispersed between lipids of a SLN. The hydrophobic moiety may be a lipid naturally existing in a biomembrane or a lipid that does not naturally exist in a biomembrane.

The lipid naturally existing in a biomembrane may be one selected from a phospholipid or its derivative, a sterol or its derivative, a sphingolipid or its derivative, and a combination thereof. The phospholipid or its derivative may be one selected from the group consisting of a phosphatidyl choline, a phosphatidyl glycerol, a phosphatidyl inositol, a phosphatidyl ethanolamine, and a combination thereof. The sterol or its derivative may be a cholesterol or its derivative, or a squalene or its derivative. The sphingolipid may be a sphingomyelin or its derivative, or a ganglioside or its derivative. The phospholipid, sterol, or sphingolipid includes an intermediate or a precursor produced during a synthesis process in vivo. For example, the hydrophobic moiety includes a phosphoglyceride, a sphingosine, a ceramide, or a cerebroside. The derivative may be an ester of a fatty acid. The fatty acid may be a fatty acid having 4 to 30 carbon atoms.

The hydrophobic moiety may be a saturated or unsaturated hydrocarbon group, a substituted amide group with the formula -C(O)N(R1)(R2) wherein R1 and R2 are each independently a saturated or unsaturated hydrocarbon group, a saturated or unsaturated acyl group, or a saturated or unsaturated alkoxy group.

A conjugation of a hydrophobic moiety and an ELP may be performed by a non-cleavable linkage under physiological and pathological conditions or by a cleavable linkage. An example of the cleavable linkage may be a linkage mediated by a pH cleavable linker, a heat cleavable linker, a radiation cleavable linker, or a linker that is cleaved in an aqueous solution.

The hydrophobic moiety may be conjugated to the ELP by being conjugated with an amino (NH₂-, or -NH-) group at a N-terminus or a carbonyl (-C(O)-) group at a C-terminus. The hydrophobic moiety may be conjugated by interaction with a functional group selected from the group consisting of an amino group, a carbonyl group, a hydroxyl group, a thiol group, and a combination thereof on a side chain of the ELP. The hydrophobic moiety may be conjugated to the ELP by an amine bond or amide bond with the nitrogen atom of the ELP. The hydrophobic moiety may be conjugated to the ELP by an amide or ester bond with the carbonyl group at the C-terminus of the ELP.

The hydrophobic region of the hydrophobic moiety or R1 and R2 of the substituted amide group with the formula -C(O)N(R1)(R2) may have 4 to 30 carbon atoms, for example, 14 to 24 carbon atoms or 16 to 24 carbon atoms. The hydrophobic moiety may be, for example, myristoyl (C14), palmitoyl (C16), stearoyl (C18), arachidonyl (C20), behenoyl (C22), or lignoceroyl (C24), or myristyl (C14), palmityl (C16), stearyl (C18), arachidyl (C20), behenyl (C22), or lignoceryl (C24). R1 and R2 of the substituted amide group with the formula -C(O)N(R1)(R2) may be, for example, myristyl (C14), palmityl (C16), stearyl (C18), arachidyl (C20), behenyl (C22), or lignoceryl (C24). The hydrophobic moiety may be packed in a lipid by a hydrophobic effect, and accordingly, the ELP conjugated to the hydrophobic moiety may be immobilized on the SLN.

An example of the ELP conjugated to a hydrophobic moiety may be a stearoyl- or cholesteryl-V'n-NH₂, where n is 1 to 200. Here, V' may represent one selected from the group consisting of VPGXG, PGXGV, GXGVP, XGVPG, and GVPGX, and when n is 2 or greater, V' included in the stearoyl- or cholesteryl-V'n-NH₂ may be same or different from each other, n may be an integer of 2 to 200, 2 to 80, 2 to 60, 2 to 40, 2 to 10, 2 to 12, 2 to 8, 2 to 6, 4 to 100, 8 to 80, 10 to 60, 12 to 40, 20 to 40, 4 to 10, 4 to 8, or 4 to 6. Here, V may be valine, P may be proline, G may be glycine, and X may be any natural or non-natural amino acid except proline. Here, X in each V' unit may be the same or a different amino acid. The ELP conjugated to a hydrophobic moiety may be, for example, a stearoyl-(VPGVG)n-NH₂ or cholesteryl-(VPGVG)n-NH₂.

Another examples of the ELP conjugated to a hydrophobic moiety may be a stearoyl- or cholesteryl-[V₁n₁V₂n₂]n₃-NH₂, where n₁, n₂, and n₃ are each independently 1 to 200. n₁, n₂, and n₃ may be each independently an integer of 2 to 200, 2 to 100, 2 to 80, 2 to 60, 2 to 40, 2 to 10, 2 to 12, 2 to 8, 2 to 6, 4 to 100, 8 to 80, 10 to 60, 12 to 40, 20 to 40, 4 to 10, 4 to 8, or 4 to 6. Here, V₁ and V₂ may each independently represent one selected from the group consisting of VPGXG, PGXGV, GXGVP, XGVPG, and GVPGX. Here, V may be valine, P may be proline, G may be glycine, and X may be any natural or non-natural amino acid except proline. Here, X in each V₁ unit may be the same or a different amino acid and X in each V₂ unit may be the same or a different amino acid. The ELP conjugated to a hydrophobic moiety may be, for example, a stearoyl- or cholesteryl-[(VPGVG)n₁(VPGAG)n₂]n₃-NH₂.

Another examples of the ELP conjugated to a hydrophobic moiety may be a stearoyl- or cholesteryl-[B(SA or Chol)n₁V₁n₂]n₃-NH₂, where n₁, n₂, and n₃ are each independently 1 to 200. n₁, n₂, and n₃ may be each independently an integer of 2 to 200, 2 to 100, 2 to 80, 2 to 60, 2 to 40, 2 to 10, 2 to 12, 2 to 8, 2 to 6, 4 to 100, 8 to 80, 10 to 60, 12 to 40, 20 to 40, 4 to 10, 4 to 8, or 4 to 6. Here, B(SA or Chol) may each independently represent one selected from the group consisting of VPGXG, PGXGV, GXGVP, XGVPG, and GVPGX. Here, V may be valine, P may be proline, G may be glycine, and X may be lysine, arginine, or histidine having a side chain amino group conjugated with a stearoyl or cholesterylmoity. Here, X in each B(SA or Chol) unit may be the same or a different amino acid and X in each V₁ unit may be the same or a different amino acid. The ELP conjugated to a hydrophobic moiety may be, for example, a stearoyl- or cholesteryl-[(VPGVG)n₁(VPGAG)n₂]n₃-NH₂. V₁ may represent one selected from the group consisting of VPGXG, PGXGV, GXGVP, XGVPG, and GVPGX, where V may be valine, P may be proline, G may be glycine, and X may be any natural or non-natural amino acid except proline. Here, when n₁ or n₂ is 2 or greater, B of each location may be same or different from each other and V₁ of each location may be same or different from each other. The ELP conjugated to a hydrophobic moiety may be, for example, a stearoyl- or cholesteryl-[(VPGK(SA or Chol)G)n₁(VPGXG)n₂]n₃-NH₂.

The ELP may be included with a ratio of 0.01 to 50 weight % (wt %) to a total weight of the SLN.

As used herein the term "lipid" includes a fat or a fat-derived substance that is relatively insoluble in water but soluble in an organic solvent. The lipid includes a fatty acid ester, a fatty alcohol, a sterol, or a wax. An example of the fat is a glyceryl ester of a higher fatty acid.

The lipid includes a neutral lipid, an amphipathic lipid, or a combination thereof. The neutral lipid refers to a lipid that is free of charge. The neutral lipid includes a glyceryl ester of a fatty acid. The neutral lipid includes a monoglyceride, diglyceirde, or triglyceride of at least one C4 to C24 carboxylic acid. The carboxylic acid may be saturated or unsaturated and may be branched or unbranched. For example, the lipid may be a monoglyceride of C4, C5, C6, C7, C8, C9, C10, C11, C12 C13,C14, C15, C16, C17, C18, C19, C20, C21, C22, C23, or C24 carboxylic acid. The carboxylic acid may be saturated or unsaturated and branched or unbranched. The carboxylic acid may be covalently linked to any one of the three glycerol hydroxyl groups. According to another embodiment, the lipid may be a diglyceride of C4, C5, C6, C7, C8, C9, C10, C11, C12, C13, C14, C15, C16, C17, C18, C19, C20, C21, C22, C23, or C24 carboxylic acids. The carboxylic acid may be saturated or unsaturated and branched or unbranched. The two carboxylic acids may be the same or different, and the carboxylic acid may be covalently linked to any two of the three glycerol hydroxyl groups. According to another embodiment, the lipid may be a triglyceride of C4, C5, C6, C7, C8, C9, C10, C11, C12, C13, C14, C15, C16, C17, C18, C19, C20, C21, C22, C23, or C24 carboxylic acids. The carboxylic acid may be saturated or unsaturated and branched or unbranched. The three carboxylic acids may be the same, two of the carboxylic acid may be the same, or all three may be different. The carboxylic acids may be covalently linked to any three of the three glycerol hydroxyl groups.

According to an embodiment, the lipid may be a blend of triglycerides of saturated, even-numbered, unbranched fatty acids with a chain length of C8 to C18. For example, the lipid may be a blend of triglycerides, each triglyceride being that including C8, C10, C12, C14, C16, or C18 carboxylic acids. For example, the lipid may be a blend of a tricapric acid glycerol and a trilauric acid glycerol. For each triglyceride in the blend, the three carboxylic acids may be the same, two of the carboxylic acid may be the same, or all three may be different.

The lipid may include a blend of monoglycerides, diglycerides, and triglycerides. The carboxylic acids of each monoglyceride, diglyceride, and triglyceride may be saturated or unsaturated, may be branched or unbranched, and may be a C4, C5, C6, C7, C8, C9, C10, C11, C12, C13, C14, C15, C16, C17, C18, C19, C20, C21, C22, C23, C24 carboxylic acid. The lipid may be a blend of monoglycerides, diglycerides, and triglycerides of saturated, even-numbered, unbranched fatty acids with a chain length of C8 to C18. For example, the lipid may be a blend of triglycerides, each triglyceride being that including C8, C10, C12, C14, C16, or C18 carboxylic acids.

The amphipathic lipid may include a hydrophilic region and a hydrophobic region. The amphipathic lipid may be a molecule having a hydrophilic head and hydrophobic tails. The amphipathic lipid may include one or more selected from the group consisting of a phospholipid, a fatty acid, and a combination thereof. The lipid may have carbon atoms of 14 to 50. The lipid may be a phospholipid. The phospholipid may have carbon atoms of 12 to 24. The phospholipid may be at least one selected from the group consisting of phosphatidyl cholines, phosphatidyl glycerols, phoaphatidyl inositols, phosphatidyl ethanolamines and a combination thereof, wherein the at least one phospholipids have one acyl group. Also, the phospholipid may have a phase transition temperature of about 10°C to about 70°C, for example, about 39°C to about 60°C, or about 38°C to about 45°C. The acyl group of the phospholipid may be saturated or unsaturated. The phospholipid may be a mixture of two or more phospholipids. A SLN having various phase transition temperatures may be produced due to the mixture of two or more phospholipids.

A phospholipid may have two acyl groups, for example, one selected from the group consisting of C12 saturated chain phospholipid (Tc= about 10°C), a C14 saturated chain phospholipid (Tc= about 24°C), a C16 saturated chain phospholipid (Tc= about 41 °C), a C18 saturated chain phospholipid (Tc=about 55°C), a C20 saturated chain phospholipid (Tc= about 65°C), a C22 saturated chain phospholipid (Tc= about 70°C), and a combination thereof. Similarly, other common phospholipids that may be used include a phosphatidyl glycerol, a phosphatidyl inositol, a phosphatidyl ethanolamine, a sphingomyelin, and a ganglioside that, have phase transition temperatures that vary in a similar fashion dependent on their acyl chain length. The phosphatidycoholine may be an egg phosphatidylcholine (PC).

An example of the C16 saturated chain phospholipid may be dipalmitoylphosphatidylcholine (DPPC). DPPC is a saturated chain (C16) phospholipid with a bilayer transition temperature of about 41.5°C. An example of the C18 saturated chain phospholipid may be 1,2-distearoyl-sn-glycero-3-phosphocholine (DSPC). DSPC is a saturated chain (C18) phospholipid with a bilayer transition temperature of about 55.10°C.

The lipid may include membrane-forming materials other than phospholipids. Exemplary materials which may form a solid-phase membrane include bola lipids or bacterial lipids. Additionally, block copolymers including a water-soluble polymer (e.g., polyethylene glycol) and a water-insoluble polymer (e.g., polypropylene oxide and polyethylethylene) may be employed. Also, the lipid includes a polymeric lipid. For example, the lipid may be esterified poly(acrylic acid) or esterified poly(vinyl alcohol).

The lipid or a mixture of the lipid may be included at a ratio of 0.01 to 90 wt % (0.5, 1, 5, 10, 15, 20, 25, 30, 35, 40, 45, 50, 55, 60, 65, 70, 75, 80, or 85 wt %), for example, 0.5-90wt%, 1.0-90wt%, 5.0-90wt%, 10.0-90wt% ,15.0-90wt%, 30.0-90wt%, 50.0-90wt%, 75.0-90wt%, 80.0-90wt%, or 10.0-80wt% to a total weight of SLNs.

The term "solid" refers to at least a portion of the SLNs being solid at room temperature and atmospheric pressure. However, the SLNs may include liquid lipid and/or entrapped solvent.

The SLN may further include a stabilizing agent thereof. The stabilizing agent may be one selected from the group consisting of a sterol or its derivative, a sphingolipid or its derivative, and a combination thereof. The stabilizing agent may be one selected from the group consisting of a cholesterol, a β- cholesterol, a fatty acid ester of cholesterol, for example, a fatty acid ester having 10 to 24 carbon atoms, a sitosterol, an ergosterol, a stigmasterol, a 4,22-stigmastadien-3-one, a stigmasterol acetate, a lanosterol, a cycloartenol, and a combination thereof.

The SLN stabilizing agent may be one selected from the group consisting of a steroid or its derivative, a sphingolipid or its derivative, and a combination thereof. The stabilizing agent may be a steroid with a property enabling incorporation into a lipid. As used herein, the term "steroid" indicates a type of organic compound including a core of gonane or a skeleton derived therefrom that contains a specific arrangement of four cycloalkane rings that are joined to each other, in other words, three cyclohexane rings designated as rings A, B, and C from left to right, and one cyclopentane ring (the D ring). Here, "a skeleton derived therefrom" includes an unsaturated bond inserted in the gonane skeleton. The steroid may vary in terms of the functional groups attached to the four ring core and the oxidation state of the rings. For example, the steroid may include a hydrophilic functional group on the rings. For example, the steroid may have a hydroxyl group. The steroid may be a sterol. The term "sterol" is a type of steroid which has the hydroxyl group at position C-3 and has a skeleton derived from cholestane. Here, the term "derived skeleton" includes an unsaturated bond inserted in the cholestane skeleton. The steroid includes a steroid found in plants, animals, and fungi. For example, all steroids may be made in cells either from lanosterol as in animals and fungi, or from cycloartenol as in plants. The sterol includes a cholesterol or its derivative. Here, "derivative" means a derivate of cholesterol which maintains a property to be inserted in a lipid bilayer. The derivative includes a fatty acid ester. The stabilizing agent may be one selected from the group consisting of a cholesterol, a sitosterol, an ergosterol, a stigmasterol, a 4,22-stigmastadien-3-one, a stigmasterol acetate, a lanosterol, a cycloartenol, and a combination thereof.

The stabilizing agent may be included at a ratio of about 0 to about 50 wt %, for example, about 1 to about 50 wt %, about 5 to about 50 wt %, about 10 to about 50 wt %, about 20 to about 50 wt %, about 30 to about 50 wt %, about 1 to about 40 wt %, about 5 to about 20 wt %, about 10 to about 40 wt %, about 20 to about 30 wt %, or about 1 to about 10 wt %, to a total weight of the SLN.

The SLN may further include a phospholipid derivative derivatized with a hydrophilic polymer. The hydrophilic polymer may be selected from a polyethylene glycol (PEG), a polylactic acid, a polyglycolic acid, a copolymer of a polylactic acid and a polyglycolic acid, a polyvinyl alcohol, a polyvinyl pyrrolidone, an oligosaccharide and a combination thereof. The derivative may be a phospholipid of C4 to C30, for example C16 to C24, conjugated with a PEG. The derivative may be a DPPC-PEG or a DSPE-PEG. The PEG may have a molecular weight of about 180 to about 50,000 Da. The derivative may be included at a ratio of about 0 to about 10 wt %, for example, about 1 to about 10 wt %, about 2 to about 10 wt %, about 3 to about 10 wt %, about 5 to about 10 wt %, about 1 to about 8 wt %, about 2 to about 5 wt %, or about 1 to about 5 wt %, to a total weight of the SLN.

The lipid constructing the SLN may have a phase transition temperature within a range from about 39°C to about 60°C. The ELP may have a phase transition temperature within a range from about 35°C to about 70°C or from about 39°C to about 70°C.

An average diameter of the SLNs may be about 10 nm to about 1500 nm, for example, about 10 nm to about 1000 nm, about 10 nm to about 500 nm, about 10 nm to about 300 nm, about 100 nm to about 300 nm, or about 100 nm to about 200 nm.

The SLN may further include an active agent. The active agent may include one selected from the group consisting of a physiologically active agent, a pharmaceutically active agent, a magnetically active agent, an imaging agent, and a combination thereof. The pharmaceutically active agent may be selected from the group consisting of anesthetic, antihistamine, antineoplastic, anti-ulcerative, anti-seizure agent, muscle relaxant, immunosuppressive agent, anti-infective agent, non-steroidal anti-inflammatory agent, imaging agent, nutritional agent, and a combination thereof. The active agent may be selected from the group methotrexate, doxorubicin, epirubicin, daunorubicin, vincristine, vinblastine, etoposide, ellipticine, camptothecin, paclitaxel, docetaxel, cisplatin, prednisone, methyl-prednisone, ibuprofen and combinations thereof. The active agent may be dispersed, embedded, or incorporated in the SLN. The active agent may be included at a ratio of about 0.01 to about 10 wt %, for example, about 0.1 to about 10 wt%, about 1 to about 10 wt %, about 3 to about 10 wt %, about 5 to about 10 wt %, about 0.01 to about 8 wt %, about 0.01 to about 5 wt %, or about 0.1 to about 5 wt %, to a total wt of the SLN.

The SLN may further include a surfactant. A surfactant is a substance that is dissolved in liquid and serves to significantly reduce a surface tension, and the surfactant is present in a molecule as divided into a hydrophilic region and a hydrophobic region. In this regard, a surfactant may conveniently adhere to a surface and form a molecular aggregate, which is a micelle, at a certain concentration (a critical micelle concentration) or higher. A surfactant may be used to inhibit lipid coagulation and enhance uniform dispersion in the current system. The surfactant may be a polyether, for example, a polyoxyethylene derivative of sorbitan monolaurate (Tween). The surfactant may be Tween 20 or Tween 80. The surfactant may be included at a ratio of about 0 to about 10 volume %, for example, about 1 to about 10 volume %, about 3 to about 10 volume %, about 5 to about 10 volume %, about 1 to about 8 volume %, about 1 to about 5 volume %, or about 3 to about 5 volume %, to a total volume of the SLN. One or more surfactants may be dissolved or suspended in an organic phase or an aqueous phase during manufacture of the SLN. According to another embodiment, one or more surfactants may be added to a suspension of the SLN after manufacturing the SLN.

When a surfactant is present in an organic phase or an aqueous phase, the surfactant may be located in an interior of the SLN (that is, encapsulated) or on an exterior of the SLN (that is, covalently or non-covalently bonded to a functional group that is present on an exterior). When one or more surfactants are mixed after forming the SLN, the surfactants may be located on the exterior of the SLN, that is located by being covalently or non-covalently bonded to the functional group present on the exterior.

According to an embodiment of the SLN, the SLN may include an ELP conjugated to a hydrophobic moiety, a first lipid, a second lipid, and a stabilizing agent, wherein the first lipid is a phospholipid, and the second lipid is a neutral lipid. The moiety, the stabilizing agent, the phospholipid, and the neutral lipid are as described above. According to an embodiment, the phospholipid may be at least one selected from the group consisting of a phosphatidyl choline, a phosphatidyl glycerol, a phoaphatidyl inositol, a phosphatidyl ethanolamine, and a combination thereof. The phospholipid may have an acyl group having 16 to 24 carbon atoms. The neutral lipid may include at least one selected from the group consisting of a monoglyceride, a diglyceride, a triglyceride of carboxylic acids having 4 to 24 carbon atoms, and a combination thereof. The stabilizing agent may be selected from the group consisting of a sterol or its derivative, a sphingolipid or its derivative, and a combination thereof. The stabilizing agent may be one selected from the group consisting of a cholesterol, a β-cholesterol, a fatty acid ester of cholesterol, for example, a fatty acid ester having 10 to 24 carbon atoms, a sitosterol, an ergosterol, a stigmasterol, a 4,22-stigmastadien-3-one, a stigmasterol acetate, a lanosterol, a cycloartenol, and a combination thereof.

According to an embodiment, the SLN may include the ELP conjugated to a hydrophobic moiety, a phosphatidylcholine, and a triglyceride composed of a tricaprin and a trilaurin, and a cholesteryl oleate. A molar ratio of a phosphatidylcholine; a tricaprin and a trilaurin; a cholesteryl oleate may be about 2 to about 5: about 0.1 to about 3: 0 to about 1. A molar ratio of the tricaprin and the trilaurin may be about 1: about 0.25 to about 4. The content of the ELP conjugated to a hydrophobic moiety may be about 0.01 to about 50wt% of phosphatidylcholine. An example of the ELP conjugated to a hydrophobic moiety may be a stearoyl- or cholesteryl-V'n-NH₂, where n is 1 to 200. Here, V' may represent one selected from the group consisting of VPGXG, PGXGV, GXGVP, XGVPG, and GVPGX, and when n is 2 or greater, V' included in each location of the stearoyl- or cholesteryl-V'n-NH₂ may be same or different from each other. n may be an integer of 2 to 200, 2 to 80, 2 to 60, 2 to 40, 2 to 10, 2 to 12, 2 to 8, 2 to 6, 4 to 100, 8 to 80, 10 to 60, 12 to 40, 20 to 40, 4 to 10, 4 to 8, or 4 to 6. Here, V may be valine, P may be proline, G may be glycine, and X may be any natural or non-natural amino acid except proline. Here, X of V' in each location may be the same or a different amino acid. The ELP conjugated to a hydrophobic moiety may be, for example, a stearoyl-(VPGVG)n-NH₂ or cholesteryl-(VPGVG)n-NH₂.

Another examples of the ELP conjugated to a hydrophobic moiety may be a stearoyl- or cholesteryl-[V₁n₁V₂n₂]n₃-NH₂. n₁, n₂, and n₃ may be each independently an integer of 2 to 200, 2 to 100, 2 to 80, 2 to 60, 2 to 40, 2 to 10, 2 to 12, 2 to 8, 2 to 6, 4 to 100, 8 to 80, 10 to 60, 12 to 40, 20 to 40, 4 to 10, 4 to 8, or 4 to 6. When n₁ and n₂ are each independently 2 or greater, V₁ of each location in the stearoyl- or cholesteryl-[V₁n₁V₂n₂]n₃-NH₂ may be same or different from each other and V₂ of each location in the stearoyl- or cholesteryl-[V₁n₁V₂n₂]n₃-NH₂ may be same or different from each other. Here, V₁ and V₂ may each independently represent one selected from the group consisting of VPGXG, PGXGV, GXGVP, XGVPG, and GVPGX. Here, V may be valine, P may be proline, G may be glycine, and X may be any natural or non-natural amino acid except proline. Here, X of V₁ in each location may be the same or a different amino acid and X of V₂ in each location may be the same or a different amino acid. The ELP conjugated to a hydrophobic moiety may be, for example, a stearoyl- or cholesteryl -[(VPGVG)n₁(VPGAG)n₂]n₃-NH₂.

Another examples of the ELP conjugated to a hydrophobic moiety may be a stearoyl- or cholesteryl-[B(SA or Chol)n₁V₁n₂]n₃-NH₂, where n₁, n₂, and n₃ may be each independently an integer of 2 to 200, 2 to 100, 2 to 80, 2 to 60, 2 to 40, 2 to 10, 2 to 12, 2 to 8, 2 to 6, 4 to 100, 8 to 80, 10 to 60, 12 to 40, 20 to 40, 4 to 10, 4 to 8, or 4 to 6. Here, B(SA or Chol) may each independently represent one selected from the group consisting of VPGXG, PGXGV, GXGVP, XGVPG, and GVPGX. Here, V may be valine, P may be proline, G may be glycine, and X may be lysine, arginine, or histidine having a side chain amino group conjugated with a stearoyl or cholesteryl moiety. V₁ may represent one selected from the group consisting of VPGXG, PGXGV, GXGVP, XGVPG, and GVPGX, where V may be valine, P may be proline, G may be glycine, and X may be any natural or non-natural amino acid except proline. Here, when n₁ or n₂ is 2 or greater, B of each location may be same or different from each other and and V₁ of each location may be same or different from each other. The ELP conjugated to a hydrophobic moiety may be, for example, a stearoyl- or cholesteryl-[(VPGK(SA or Chol)G)n₁(VPGXG)n₂]n₃-NH₂.

A method of manufacturing a SLN is known. The SLN may be manufactured by a melting/solidification process, wherein a lipid is first melted, dispersed in water and then cooled to solidify the SLN. Also, the SLN may be produced using an emulsion process akin to the formation of polymeric microparticles, wherein the lipids are dissolved in a solvent, emulsified, and then dispersed in an aqueous solution containing an emulsifying agent to harden the SLN. The role of the emulsifying agent is to stabilize the SLN. Alternatively, the SLN may be manufactured by a film formation/hydration process, wherein a lipid and a hydrophobic drug are melted in and removed from an organic solvent at the same time, and then through sonication and vortexing, the SLN inserted with the drug may be manufactured. According to a composition of the SLN, the methods above may be separately used.

A SLN may be manufactured by, for example, a method including providing an organic phase including an ELP, a lipid, and a binary solvent system; producing a thin film by removing the organic phase; providing an aqueous phase including water; and combining and dispersing the thin film and the aqueous phase. The organic phase or a part of the organic phase may be selectively removed, and accordingly the SLN may be manufactured as an aqueous suspension. The term "binary solvent system" refers to a solvent system including two or more of miscible or partially miscible solvents. The term particularly includes a three-solvent, a 4-solvent, and a 5-solvent system. The solvent system generally includes solvents that are liquid at room temperature and atmospheric pressure. However, although an entire system is liquid at room temperature and atmospheric pressure, one or more solvents of the system needs to be understood as being solid or gaseous at room temperature and atmospheric pressure. The binary solvent system may be chloroform(CHCl₃)-ethanol, choloroform(CHCl₃)-methanol, dichloromethane(CH₂Cl₂)-ethanol, dichloromethane(CH₂Cl₂)-methanol, N-methylpyrrolidone(NMP)-acetone, tetrahydrofuran (THF)-acetone, or dimethylformamide(DMF)-acetone.

SLN may be also manufactured by a method including providing an organic phase including an ELP, a neutral lipid, and a binary solvent system; producing a thin film by removing the organic phase; providing an aqueous phase including water; and combining and dispersing the thin film and the aqueous phase. However, a known method may be used for manufacturing of SLN, and is not limited to a particular method.

According to an embodiment, the SLN may include an ELP conjugated to a hydrophobic moiety, a first lipid, a second lipid, and a stabilizing agent, wherein the first lipid is a phospholipid, and the second lipid is a neutral lipid, wherein the SLN may have a structure of the second lipid and the stabilizing agent forming an inner core, and the first lipid surrounding the inner core. The first lipid may surround the inner core in a form of a monolayer. The ELP may be immobilized on the SLN due to interaction with the first lipid and optionally the second lipid via the moiety. The ELP, the first lipid, the second lipid, and the stabilizing agent are as described above. Also, the SLN may include a surfactant.

According to another aspect of the present invention, a pharmaceutical composition for delivering an active agent to a target site in a subject includes pharmaceutically acceptable carriers or diluents, and a SLN of the present invention containing the active agent, wherein, the SLN includes an ELP conjugated to a hydrophobic moiety and lipids.

The pharmaceutically acceptable carrier or diluent may be well known in the art. The carrier or diluent may be selected from the group consisting of water, for example saline or sterile water, Ringer's solution, buffered saline, dextrose solution, maltodextrose solution, glycerol, ethanol, and combinations thereof.

The SLN has already been described above.

The SLN may be dispersed in an aqueous medium. The aqueous medium may include physiological saline or PBS. Also, the SLN may be entrapped within a liposome or formulated in a form of a dispersion or an emulsion. The active agent may be entrapped in the interior space of the SLN. The active agent may be entrapped in the lipid molecules of the SLN. The SLN may have a phase transition temperature of about 39°C to about 45°C.

The active agent may include one or more selected from the group consisting of a physiologically active agent, a pharmaceutically active agent, a magnetically active agent, an imaging agent, and a combination thereof. The active agent may be one or more of a water-insoluble component, a water-soluble component, and a combination thereof. The pharmaceutically active agent may be selected from the group consisting of anesthetic, antihistamine, antineoplastic, anti-ulcerative, anti-seizure agent, muscle relaxant, immunosuppressive agent, anti-infective agent, non-steroidal anti-inflammatory agent, imaging agent, nutritional agent, and a combination thereof. The active agent may be selected from the group methotrexate, doxorubicin, epirubicin, daunorubicin, vincristine, vinblastine, etoposide, ellipticine, camptothecin, paclitaxel, docetaxel, cisplatin, prednisone, methyl-prednisone, ibuprofen and a combination thereof.

A method of delivering an active agent to a target site in a subject includes administrating SLN containing the active agent to a subject, wherein SLN includes an ELP conjugated to a hydrophobic moiety, and lipids; and heating the target site of a subject to release the active agent from the SLN at the target site. For releasing the active agent, the SLN is preferably heated above its phase transition temperature and/or above the phase transition temperature of at least one of its ELPs.

The method includes administrating the SLN containing the active agent to the subject. The SLN containing the active agent have already been described above. The SLN may have a phase transition temperature of from about 39°C to about 45°C.

The administration may be parenteral administration. The parenteral administration, for example, may be intravenous, intradermal, intramuscular, intracavity (abdominal cavity, joints, or eye), or direct injection. The direct injection may involve injecting directly into a diseased site such as a tumor site. The SLN may be administered intravenously and thereby brought to the target site such as a tumor site by blood flow. The target site may have a leaky property. The term "leaky property" indicates characteristics of the target site with increased permeability compared to normal tissues or cells. The target site may be a tumor site where a material permeability of blood vessels in the tumor is increased due to increased leakiness of the tumor blood vessels.

The subject may be a human or a mammal excluding a human. The mammal may be selected from the group consisting of a dog, a cat, a horse, a cow, a pig, a goat, a monkey, a mouse, and a combination thereof.

The method includes heating the target site of the subject to release the active agent from the SLN at the target site. The heating may be due to a clinical procedure that induces hyperthermia or may be related to an intrinsically higher temperature of an inflamed body part compared to the rest of the body. The clinical procedure that induces hyperthermia may be performed by direct heat transfer, for example, a hot liquid medium in a tub, e.g., contacting a body in water, irradiating ultrasound, e.g., high intensity ultrasound focused at a target site, applying a magnetic field, e.g., an amplified magnetic field, applying microwave energy and/or high frequency energy. The target site may be a region where pathological symptoms exist, for example, a tumor site (e.g., a solid tumor), or where inflammation exists. The heating may involve heating to a temperature of about 39°C to about 45°C.

The active agent may include one or more selected from the group consisting of a physiologically active agent, a pharmaceutically active agent, a magnetically active agent, an imaging agent, and a combination thereof. The pharmaceutically active agent may be selected from the group consisting of an anesthetic, antihistamine, antineoplastic, anti-ulcerative, anti-seizure agent, muscle relaxant, immunosuppressive agent, anti-infective agent, non-steroidal anti-inflammatory agent, imaging agent, nutritional agent, and a combination thereof. The active agent may be selected from the group methotrexate, doxorubicin, epirubicin, daunorubicin, vincristine, vinblastine, etoposide, ellipticine, camptothecin, paclitaxel, docetaxel, cisplatin, prednisone, methyl-prednisone, ibuprofen and combinations thereof

According to the SLN of an embodiment, a dispersibility of particles may be adjusted by shrinking and self-assembling of an ELP conjugated to a hydrophobic moiety depending on a temperature. Therefore, the SLN may be used as a vehicle for effectively delivering an active agent to a target site of a subject.

According to the SLN including the active agent, the dispersibility of the particles and a releasability of the active agent may be adjusted by a phase transition temperature of ELP conjugated to a hydrophobic moiety as well as a phase transition temperature of SLN itself. Thus, when the SLN has a more stable composition at body temperature, for example, even at a status containing an effective amount of molecules, such as cholesterols, which stabilize lipids for maintaining SLN more stably at body temperature, the dispersibility of the particles and the releasability of the active agent may be efficiently adjusted by the phase transition temperature of ELP conjugated to a hydrophobic moiety.

According to a pharmaceutical composition for delivering an active agent containing the SLN according to another embodiment to a subject, the composition may be used to efficiently deliver the active agent to the subject.

According to a method of administering the active agent to the target site in the body of the subject according to another embodiment, the active agent may be efficiently delivered to the target site in the body of the subject.

The present invention will now be described more fully with respect to exemplary embodiments.

### Example 1: Preparation of SLN including sparingly water-soluble methyl red

A SLN was prepared using lipids. An egg phosphatidylcholine (PC) as phospholipids, and a mixture of tricaprin and trilaurin (at a molar ratio of tricaprin : trilaurin is 6:4) as a triglyceride, and cholesteryl oleate as a stabilizing agent was dissolved in a mixed solvent of chloroform and ethanol (at a volume ratio of 2:1) at room temperature and atmospheric pressure (at a molar ratio of PC: triglyceride: cholesteryl oleate = 5:2:1). 1 to 2 ml of the resultant solution was added to a container including 1 ml of PBS and dispersed by vortexing and sonication, and an organic solvent was evaporated by using a rotary evaporator, and thus, the SLN was formed (hereinafter, referred to as "a control group SLN") in 1ml of PBS since the organic solvent was evaporated. Also, methyl red, corresponding to 10 wt % of an egg PC, was treated in the same manner described above, except dissolving the methyl red together with egg PC, cholesteryl oleate, and the mixture of tricaprin and trilaurin (at a molar ratio of 6:4) as a triglyceride, and thus MR-containing SLN was prepared (hereinafter, referred to as "a experimental group SLN").

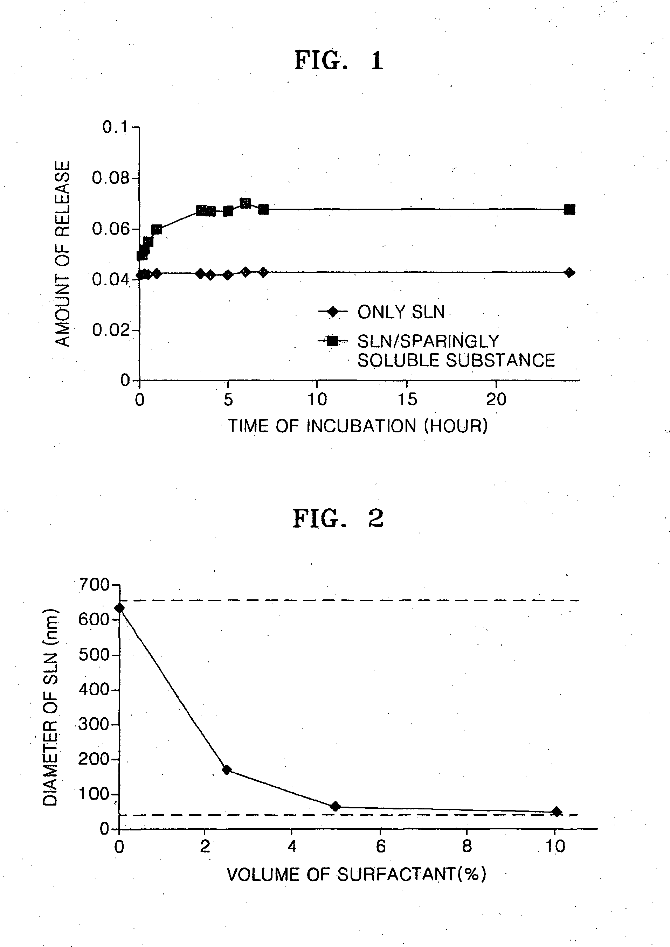

FIG. 1 shows a MR emission profile of SLN prepared in Example 1. 400 µL of the SLN (1 mg of Egg PC (main lipid)/mL of PBS, 10wt% MR of Egg PC) including a sparingly water-soluble material (the experimental group SLN) and the SLN not including a sparingly water-soluble material (the control group SLN) were added to 50 mL of PBS, and then absorbance values were measured at 410 nm, which is the max absorbance wavelength of MR, according to time while being stored at room temperature and pressure. FIG. 1 illustrates the results of the absorbance values. The horizontal axis indicates absorbance values measuring time, and the vertical axis indicates absorbance values. As shown in FIG. 1, emission of MR according to time at room temperature was insignificant (1% or less of the incorporated amount). That is, the SLN not having an ELP had a very low emission of the incorporated material.

In addition, an average diameter of the obtained SLN was measured by using Zetasizer Nano ZS (Malvern instrument, UK), which is a dynamic light scattering (DLS) device. Particularly, 10 µL of the SLN (1 mg of Egg PC/ 1 mL of PBS, 10 wt% MR of Egg PC) was added to a cuvette (Disposable solvent resistant MicroCuvette (ZEN0040), Malvern, UK) containing 90 µL of PBS, and the average diameter was measured by light scattering in Zetasizer. As a result, the average diameter of the SLN including MR was 168 nm ± 0.5 nm.

### Example 2: Preparation of SLN including surfactant

In the current embodiment, an egg PC as phospholipids, an oleate as a stabilizing agent, and a mixture of a tricaprin and a trilaurin (at a molar ratio of 6:4) as a triglyceride were dissolved in a mixed solvent of chloroform and ethanol (at a volume ratio of 2:1) at room temperature and atmospheric pressure at a molar ratio of 5:1:2 of egg PC: triglyceride: cholesteryl oleate. 1 to 2 ml of the resultant solution was added to containers each including 1 ml of PBS of a different ratio of the surfactant Tween 20 (0, 2.5, 5, or 10 vol% based on a PBS volume) and dispersed by vortexing and sonication, and an organic solvent was evaporated by using a rotary evaporator, and thus, the SLN was formed in 1ml of PBS since the organic solvent was evaporated.

An average diameter of the obtained SLN was measured by using Zetasizer Nano ZS (Malvern instrument, UK), which is a dynamic light scattering (DLS) device. Particularly, 10 µL of the SLN (1 mg of Egg PC/ 1 mL of PBS, 10 wt% MR of Egg PC) was added to a cuvette (Disposable solvent resistant MicroCuvette (ZEN0040), Malvern, UK) containing 90 µL of PBS, and the average diameter was measured by light scattering in Zetasizer. As a result, the average diameter of the produced SLN was from about 50 nm to about 650 nm.

FIG. 2 shows an average diameter of the SLN according to an amount of a surfactant prepared in Example 2. As shown in FIG. 2, the average diameter of the SLN decreased as the amount of Tween 20 increased.

### Example 3: Preparation of SLN including sparingly water-soluble substance and confirmation using transmission electron microscope

In the current embodiment, an egg PC as phospholipids, a mixture of a tricaprin and a trilaurin (at a molar ratio of 6:4) as a triglyceride, and a cholesteryl oleate were dissolved at a molar ratio of 5:2:1 in a mixed solvent of chloroform and ethanol (at a volume ratio of 2:1) at room temperature and atmospheric pressure along with coumarin-6 (10 weight % of egg PC), which is a sparingly water-soluble substance. 1 to 2 ml of the resultant solution was added to a container including 1 ml of PBS (containing 2 vol% of Tween 20 based on a PBS volume) and dispersed by vortexing and sonication, and an organic solvent was evaporated by using a rotary evaporator, and thus, the SLN was formed in 1 ml of PBS since the organic solvent was evaporated.

An average diameter of the obtained SLN was measured by using Zetasizer Nano ZS (Malvern instrument, UK), which is a dynamic light scattering (DLS) device. Particularly, 10 µL of the SLN (1 mg of Egg PC/ 1 mL of PBS, 10 wt% coumarin-6 of Egg PC) was added to a cuvette (Disposable solvent resistant MicroCuvette (ZEN0040), Malvern, UK) containing 90 µL of PBS, and the average diameter was measured by light scattering in Zetasizer. As a result, the average diameter of the produced SLN was from about 200 nm to about 220 nm. Moreover, the coumarin-6 particles introduced in the solid lipids were confirmed by a transmission electron microscope (TEM) observation.

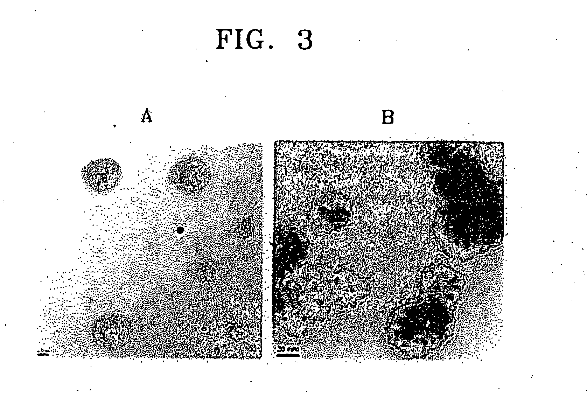

FIG. 3 showed TEM images of (A) SLN not containing coumarin-6 and (B) SLN containing coumarin-6. As shown in FIG. 3, black spots corresponding to the coumarin-6 particles were present in the SLN.

Also, a stability of the produced SLN was measured. While storing the SLN prepared in the same manner of Example 1, that is a solution having a concentration of SLN corresponding to 1 mg of Egg PC/ml of PBS (MR not included), at room temperature and atmospheric pressure for one week, a particle size was measured on the date of manufacture, after 24 hours, and after 72 hours, by using Zetasizer in the same manner described above. An average particle size was about 168 nm ± 0.5 nm which indicated that the particle size did not change even after being stored for about 72 hours.

### Example 4: Preparation of SLN according to types of lipids and amount of surfactant

In the current embodiment, a SLN was manufactured in the same manner used in Example 3, except that a type of the phospholipid was changed to egg PC or dipalmitoylphosphatidylcholine (DPPC) and a ratio of tricaprin and trilaurin as a triglyceride was changed to 8:2, and thus lipid nanoparticles were manufactured (not containing coumarin-6). Also, the amount of Tween 20 used, was 0, 1, or 2 vol% of PBS volume. A concentration of the manufactured lipid nanoparticles was 1 mg/1 mL of Tween 20-containing PBS.

An average diameter of the obtained SLN was measured by using Zetasizer Nano ZS (Malvern instrument, UK), which is a dynamic light scattering (DLS) device. Particularly, 10 µL of the SLN (1 mg of Egg PC/ 1 mL of PBS, 10 wt% coumarin-6 of Egg PC) was added to a cuvette (Disposable solvent resistant MicroCuvette (ZEN0040), Malvern, UK) containing 90 µL of PBS, and the average diameter was measured by light scattering in Zetasizer.

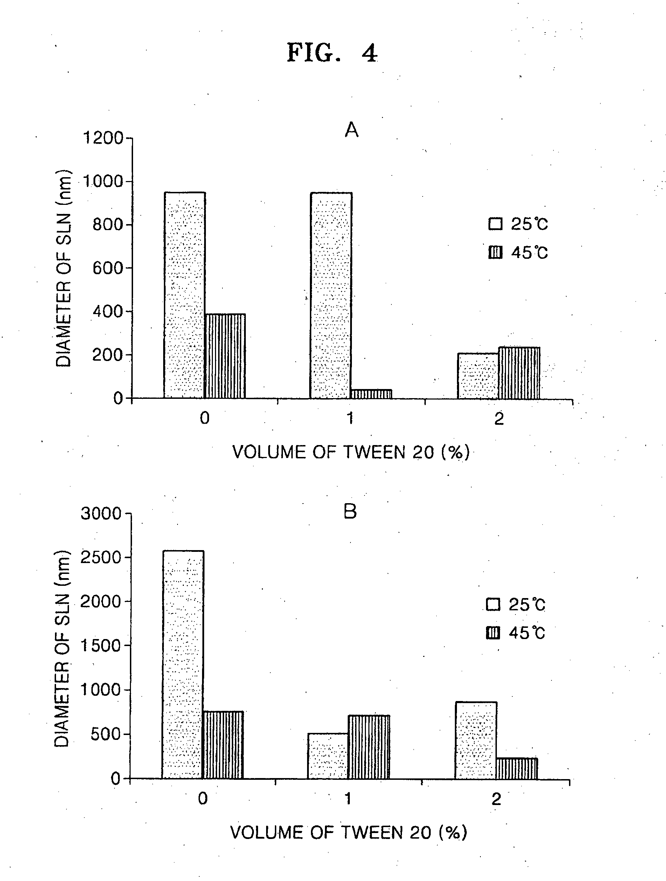

FIG. 4 shows an average diameter of the SLN according to types of lipid molecules and amounts of a surfactant. A mixture of 10 µL of SLN solution prepared in the same manner of Example 4 and 90 µL of PBS was prepared, and the average diameter was measured by using Zetasizer in the same manner described above while maintaining a temperature of the mixture between 25°C and 45°C for five minutes. As shown in FIG. 4, when the phospholipids were egg PC (Fig. 4A), the average diameter of the produced SLN was from about 200 nm to about 950 nm at a temperature of 25°C and from about 50 nm to about 400 nm at a temperature of 45°C. When the phospholipids were DPPC (Fig. 4B), the average diameter of the produced SLN was from about 500 nm to about 2580 nm at a temperature of 25°C and from about 250 nm to about 700 nm at a temperature of 45°C.

### Example 5: Preparation of SLN including ELP and Measurement of particle size

An SLN was prepared using stearoyl-(VPGVG)n-NH₂ (hereinafter referred to as "SA-Vn"), wherein n is 1 to 200, as an ELP. Here, the stearoyl group is linked to a nitrogen at an N-terminal by an amide bond.

In the current embodiment, SA-V5 and SA-V6 (available from Peptron Inc., Korea) of which n is 5 and 6 (10wt% of Egg PC), an egg PC as phospholipids, a mixture of a tricaprin and a trilaurin (at a molar ratio of 6:4) as a triglyceride, and a cholesteryl oleate were dissolved at a molar ratio of 5:2:1 (egg PC: triglyceride: cholesteryl oleate) in a mixed solvent of chloroform and ethanol (at a volume ratio of 2:1) at room temperature and atmospheric pressure. The organic solvent was evaporated by using a rotary evaporator, and thus, the lipid solution was formed as a film. Then, by adding PBS, vortexing, and sonication, a SLN solution in which particles were dispersed was obtained.

An average diameter of the obtained SLN was measured by using Zetasizer Nano ZS (Malvern instrument, UK), which is a dynamic light scattering (DLS) device. Particularly, 10 µL of the SLN (1 mg of Egg PC/ 1 mL of PBS, 10 wt% ELP of Egg PC) was added to a cuvette (Disposable solvent resistant MicroCuvette (ZEN0040), Malvern, UK) containing 90 µL of PBS, and the average diameter was measured by light scattering in Zetasizer. As a result, the average diameter of the produced SLN was 220 nm (SA-V5) or 120 nm (SA-V6) according to a length of the ELP used.

### Example 6: Preparation of SLN including ELP and Particle size change depending on temperature

A SLN was prepared using cholestearoyl-(VPGVG)n1-(VPGAC)n2-NH₂ (hereinafter referred to as "Chol-Vn1 An2"), wherein n1 and n2 (Peptron, Korea) are each independently 1 to 200, as an ELP.

In the current embodiment, Chol-Vn1An2 (0, 1, 2.5, and 5 wt % of egg PC) of which n1 is 3 and n2 is 1 (Peptron, Korea), an egg PC as phospholipids, a mixture of a tricaprin and a trilaurin (a molar ratio of 8:2) as a triglyceride, and a cholesteryl oleate were dissolved with a molar ratio of 5:2: 1 (egg PC: triglyceride: cholesteryl oleate) in a mixed solvent of chloroform and ethanol (at a volume ratio of 2:1) at room temperature and atmospheric pressure. The organic solvent was evaporated by using a rotary evaporator, and thus, the lipid solution was formed as a film. Then, by adding PBS, vortexing, and sonication, a SLN solution in which particles were dispersed was obtained.

An average diameter of the obtained SLN was measured by using Zetasizer Nano ZS (Malvern instrument, UK), which is a dynamic light scattering (DLS) device. Particularly, 10 µL of the SLN (1 mg of Egg PC/ 1 mL of PBS, 0, 1, 2.5, and 5 wt% ELP of Egg PC) was added to a cuvette (Disposable solvent resistant MicroCuvette (ZEN0040), Malvern, UK) containing 90 µL of PBS, and the average diameter was measured by light scattering in Zetasizer.

Tables 1 and 2 illustrate the average diameters of the SLN measured as described above.

**Table 1.**

| Sample name | Main peak (d.nm) | 2^{nd} peak (d.nm) | 3^{rd} peak (d.nm) | Pdl |
|---|---|---|---|---|
| w/o Chol-V3A-25°C | 1046 (100%) | - | - | 0.498 |
| 1% Chol-V3A-25°C | 326.9 (61%) | 82.92 (37%) | 5204 (2%) | 0.412 |
| 2.5% Chol-V3A-25°C | 181(90%) | 34.22 (6%) | 5194 (4%) | 0.379 |
| 5% Chol-V3A-25°C | 212.3(98%) | 5207 (2%) | | 0.4 |

**Table 2.**

| Sample name | Main peak (d.nm) | 2^{nd} peak (d.nm) | 3^{rd} peak (d.nm) | Pdl |
|---|---|---|---|---|
| w/o Chol-V3A-42°C | 134.2 (67%) | 1746 (33%) | - | 0.677 |
| 1%Chol-V3A-42°C | 1.78.4(100%) | - | - | 0.296 |
| 2.5%Chol-V3A-42°C | 148.1 (100%) | - | - | 0.138 |
| 5%Chol-V3A-42°C | 164.7 (100%) | - | - | 0.304 |

| | | | | |
|---|---|---|---|---|
| * %: percent of particles having the average diameter in a whole particle distribution **Pdi: polydispersity index *** d: diameter | | | | |

As shown in Tables 1 and 2, when the SLN included the ELP, the average diameter was about 180 nm to 330 nm at a temperature of 25°C and about 150 nm to about 180 nm at a temperature of 42°C. When the SLN did not include the ELP, the average diameter was about 1050 nm at a temperature of 25°C and about 134 nm to about 1750 nm at a temperature of 45°C. By introducing Chol-Vn1An2, which is an ELP, to the SLN, a temperature sensitivity of the particles was increased. When the SLN included an ELP, the average diameter according to temperature decreased, and a polydispersity was also improved as well. For example, a value of Pdi decreased at 45°C compared to the polydispersity at 25°C.

### Example 7: Preparation of SLN including ELP and Observation of particle size change depending on composition of core lipid and temperature

A SLN was prepared using cholesteryl-(VPGVG)n1 (VPGAG)n2-NH₂ (hereinafter referred to as "Chol-Vn1An2": VPGVG(SEQ NO: 6) and VPGAG(SEQ NO: 7)), wherein n is 1 to 200, as an ELP.

In the current embodiment, Chol-Vn1An2 (1 wt % of egg PC) of which n1 is 3 and n2 is 1, an egg PC as phospholipids, a mixture of a tricaprin and a trilaurin (at a molar ratio of 7:3) as a triglyceride, and a cholesteryl oleate were dissolved with a molar ratio of 5:2:1 or 5:2:0 in a mixed solvent of chloroform and ethanol (a volume ratio of 2:1) at room temperature and atmospheric pressure. The organic solvent was evaporated by using a rotary evaporator, and thus, the lipid solution was formed as a film. Then, by adding PBS (with or without 5 % triton X-100 based on a PBS volume: when 1 mL of PBS is added, 50 µL of triton X-1 00 is added) to the obtained SLN film, vortexing and sonication, a SLN solution in which particles were dispersed was obtained. Triton X-100 is a surfactant that has the same non-ionic properties as Tween.

An average diameter of the obtained SLN was measured by using Zetasizer Nano ZS (Malvern instrument, UK), which is a dynamic light scattering (DLS) device. Particularly, 10 µL of the SLN (1 mg of Egg PC/ 1 mL of PBS, 1 wt% ELP of Egg PC) was added to a cuvette (Disposable solvent resistant MicroCuvette (ZEN0040), Malvern, UK) containing 90 µL of PBS, and the average diameter was measured by light scattering in Zetasizer.

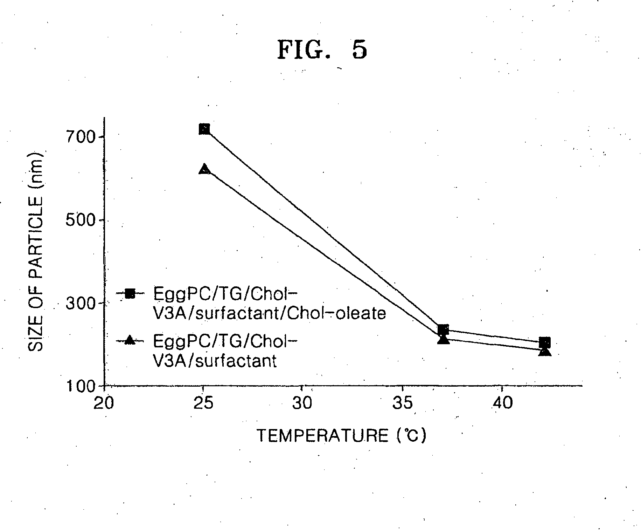

FIG. 5 shows a temperature sensitivity of particle dispersion according to a factor whether cholesteryl oleate, which performs as a stabilizing agent of the SLN, is included in the particles or not as described in Example 7. When the SLN included cholesteryl oleate, the average diameter was about 718 nm at a temperature of 25°C, about 236 nm at a temperature of 37°C, and about 206 nm at a temperature of 42°C. When the SLN did not include cholesteryl oleate, the average diameter of the SLN was about 630 nm at a temperature of 25°C, about 210 nm at a temperature of 37°C, and about 190 nm at a temperature of 42°C. It was confirmed that when cholesteryl oleate which serves as a stabilizing agent of SLN was introduced in the particles, a temperature sensitivity and dispersivity of the SLN including an ELP were increased.

### Example 8: Paclitaxel Incorporation of SLN including ELP and Confirmation of release depending on temperature

A SLN was prepared using cholestearoyl-(VPGVG)n1(VPGAG)n2-NH₂ (hereinafter referred to as "Chol-Vn1An2"), wherein n is 1 to 200, as an ELP.

In the current embodiment, Chol-Vn1An2 (1 wt % of egg PC) of which n1 is 3 and n2 is 1 (Peptron, Korea), paclitaxel (10 wt % of an amount of the egg PC), an egg PC as phospholipids, and a mixture of a tricaprin and a trilaurin (at a molar ratio of 7:3) as a triglyceride (a molar ratio of egg PC : triglyceride was 5:2) were dissolved in a mixed solvent of chloroform and ethanol (at a volume ratio of 2:1) at room temperature and atmospheric pressure. The organic solvent was evaporated by using a rotary evaporator, and thus, the lipid solution was formed as a film. Then, by adding PBS, vortexing, and sonication, a SLN solution in which particles were dispersed was obtained.

An average diameter of the obtained SLN was measured by using Zetasizer Nano ZS (Malvern instrument, UK), which is a dynamic light scattering (DLS) device. Particularly, 10 µL of the SLN (1 mg of Egg PC/ 1 mL of PBS, 10 wt% paclitaxel of Egg PC) was added to a cuvette (Disposable solvent resistant MicroCuvette (ZEN0040), Malvern, UK) containing 90 µL of PBS, and the average diameter was measured by light scattering in Zetasizer.

As a result, when the SLN including paclitaxel included Chol-V3A, the average diameter was about 720 nm at a temperature of 25°C and about 240 nm at a temperature of 42°C. When the SLN did not include Chol-V3A, the average diameter was about 1350 nm at a temperature of 25°C and about 310 nm at a temperature of 42°C. It was confirmed that when Chol-V3A was introduced in the SLN, a dispersivity of the SLN increased depending on temperature. When measured in Zetasizer, an obtained PDi value decreased, an average diameter of the measured particles in the given sample reduced, and thus these are deemed as indications of reduced size differences between particles.

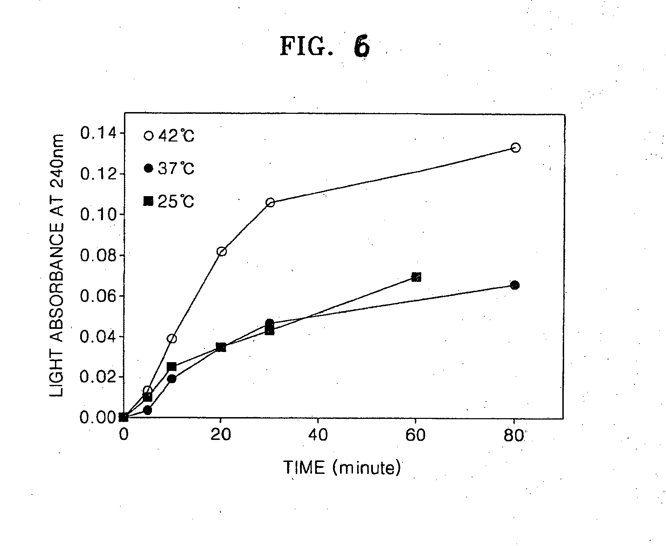

FIG. 6 shows a temperature sensitivity of drug release of the SLN including ELP, in which Paclitaxel is included as described in Example 8. As shown in FIG. 6, the drug was released rapidly at about 20 minutes at 42°C, but had a similar drug releasing tendency at 25°C and 37°C, and no rapid drug release was observed even after about 60 minutes.

FIG. 6 is the results of confirming a degree of Paclitaxel release from the SLN by using a semipermeable membrane and UV. Particularly, 2.5 mg of the SLN (5 mg of egg PC/ ml) was added to the semipermeable membrane (Spectra/PorTM Dialysis membrane, MWCO 1,000) in 25 mL of PBS medium (1 vol% Tween 20), the medium was incubated at a temperature of 25°C, 37°C, or 42°C, and a degree of absorbance of the solution outside the semipermeable membrane at a predetermined time was measured at 240 nm, which is the maximum absorbance wavelength of Paclitaxel (λₘₐₓ). As a result, the drug release was twice or more after 10 minutes at a temperature of 42°C compared to at a temperature of 25 °C and 37 °C

### Example 9: Coumarin-6 incorporation of SLN including ELP and Confirmation of cellular uptake depending on temperature

A SLN was prepared using cholesteryl-(VPGVG)n1(VPGAG)n2-NH₂ (hereinafter referred to as "Chol-Vn1An2"), wherein n is 1 to 200, as an ELP.

In the current embodiment, Chol-Vn1An2 (1 wt % of egg PC) of which n1 is 3 and n2 is 1, coumarin-6 (10 wt % of an amount of egg PC), and an egg PC as phospholipids, and a mixture of a tricaprin and a trilaurin (a molar ratio of 7:3) as a triglyceride (a molar ratio of the egg PC and the triglyceride was 5:2) were dissolved in a mixed solvent of chloroform and ethanol (a volume ratio of 2:1) at room temperature and atmospheric pressure. The organic solvent was evaporated by using a rotary evaporator, and thus, the lipid solution was formed as a film. Then, by adding PBS, vortexing, and sonication, a SLN solution in which particles were dispersed was obtained.

A degree of the cellular uptake of the obtained SLN was confirmed by analyzing using flow cytometry. The SLN incorporated with coumarin-6 was treated on a KB cell which is a cancer cell. Then, after 2 hours, a degree of the cellular uptake was measured using an intensity of fluorescence (using a FITC filter). In particular, KB cells (ATCC Number: CCL-17^{™}) are inoculated on a 12-well plate including 500 µL of growth medium (10% (v/v) FBS containing ATCC-formulated Eagle's Minimum Essential Medium) at a concentration of 2.5x10⁵ cells/well, and then the KB cells were incubated at a temperature of 37°C in a CO₂ incubator until a cell concentration reached 80% confluence. 500 µL of new growth medium was added to each well of the incubated medium, and coumarine-6 was added to the growth medium at 9 µg/mL per well. The well was stored at a temperature of 37 °C or 42 °C for 10 minutes, incubated in a 5% CO₂ incubator at a temperature of 37°C for 2 hours, and then fluorescence released from the cells were measured by performing flow cytometry by using a fluorescence-activated cell sorting (FACS) apparatus (Canto II, BD bioscience).

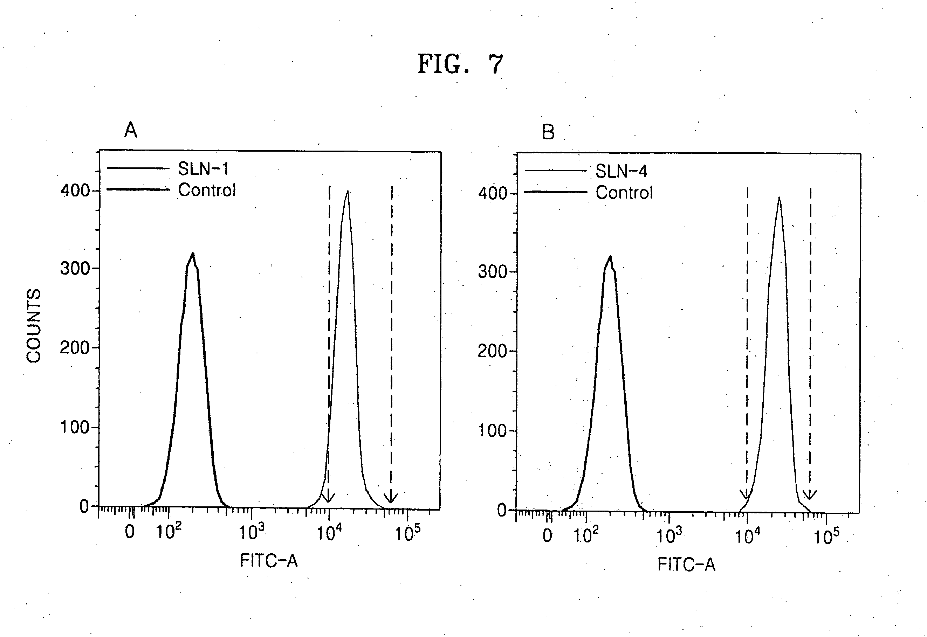

FIG. 7 illustrates the results of in-cell delivery of coumarine-6 by using the SLN in the same manner as Example 9. When the sparingly water-soluble substance, that is, coumarin-6, was incorporated in the SLN, the degree of in-cell delivery increased 100 times or more. In FIG. 7, SLN-1 indicates SLN of a composition without Chol-V3A, and SLN-4 indicates solid nanoparticles of a composition with Chol-V3A. Control indicates directly treating a cell with coumarin-6 (which was treated on a 12-well plate at a concentration of 9 µg/ml of coumarine-6 to total 4.5 µg of coumarine-6 per well). As shown in FIG. 7, it may be confirmed that the SLN-4 were internalized in more number of cells than SLN-1. In FIG. 7, the number of cells is evaluated by a value of surface area under the curve, and a range of X values of SLN-1, which is about 5,000 to about 30,000, is smaller than a range of SLN-4, which is about 9,000 to about 60,000.

### Example 10: SLN including ELP having a plurality of acyl groups

A SLN was prepared using SA-[K'(SA)V'₃]₂-NH₂ as an ELP. Here, SA is stearoyl, K' indicates VPGKG (SEQ ID NO: 8), K'(SA) indicates a stearoyl group attached to NH₂ of the side chain of lysine of VPGKG, V' may represent one selected from the group consisting of VPGXG, PGXGV, GXGVP, XGVPG, and GVPGX, and the first, second, and third V's may be the same or different sequences from one another. Here, V may be valine, P may be proline, G may be glycine, and X may be any natural or non-natural amino acid except proline. Here, X in each V' unit may be the same or a different amino acid.

In the current embodiment, SA-[(VPGK(SA)G)(VPGVG)₃]₂-NH₂ (SEQ ID NO: 9, 3 wt% of egg PC), Paclitaxel (10 wt% of egg PC), an egg PC as phospholipids, a mixture of a tricaprin and a trilaurin (at a molar ratio of 6:4) as a triglyceride , and a cholesteryl oleate as a stabilizing agent were dissolved in a mixed solvent of chloroform and ethanol (at a volume ratio of 2:1) at room temperature and atmospheric pressure at a molar ratio of 5:2:1 of egg PC: triglyceride: cholesteryl oleate. The organic solvent was evaporated by using a rotary evaporator to filmize the SLN. 1 vol% Tween 20 containing PBS was added to the SLN film, and vortexing and sonication were repeated to obtain a SLN solution where particles are dispersed (SLN 5 mg/ml). As a control group, a SLN not including cholesteryl oleate was prepared in the same manner described above.

An average diameter of the obtained SLN was measured by using Zetasizer Nano ZS (Malvern instrument, UK), which is a dynamic light scattering (DLS) device. Particularly, 10 µL of the SLN (1 mg of Egg PC/ 1 mL of PBS, 10 wt% paclitaxel of Egg PC) was added to a cuvette (Disposable solvent resistant MicroCuvette (ZEN0040), Malvern, UK) containing 90 µL of PBS, and the average diameter was measured by light scattering in Zetasizer

As a result, an average diameter of the SLN when including cholesteryl oleate (hereinafter, referred to as "an experiment group") was about 360 nm and about 310 nm at 37°C and 42 °C, respectively, and was about 270 nm and about 270 nm at 37°C and 42°C when not including cholesteryl oleate. An average diameter of the SLN including cholesteryl oleate decreased as the temperature increased above 37°C. When measured in Zetaziser, the average diameter of the SLN were measured while maintaining the temperature at 37°C and 42°C for 5 minutes.

Table 3 shows dispersity of the SLN measured according to temperature.

**[Table 3]**

| Sample name | Polydispersity index | | |
|---|---|---|---|
| | 25°C | 37°C | 42°C |
| Experimental group | 0.63 | 0.14 | 0.24 |
| Control group | 0.44 | 0.24 | 0.23 |

As shown in Table 3, polydispersity indexes of the SLN in both of the experimental group and the control group decreased as temperature increased. That is, when temperature increased, the SLN in 1 wt% Tween 20 containing PBS were more uniformly dispersed in the solution. Accordingly, Paclitaxel included in the SLN is predicted to be readily released. As dispersity increases, a large particle becomes small particles, a surface area of the SLN increases, and thus the drug included inside of the particles are exposed to the outside of the lipid particles, that is the solvent. Therefore, drug is predicted to be readily released as a size of the particles is reduced and a dispersity increases.
## Claims

1. A solid lipid nanoparticle (SLN) comprising:
an elastin-like polypeptide (ELP) conjugated to a hydrophobic moiety; and
a lipid molecule,
wherein the hydrophobic moiety is a saturated or unsaturated hydrocarbon group, a substituted amide group with the formula -C(O)N(R1)(R2) wherein R1 and R2 are independently a saturated or unsaturated hydrocarbon group, or a saturated or unsaturated alkoxygroup, wherein the lipid molecule is a neutral lipid molecule or an amphipathic lipid molecule, and wherein the ELP comprises at least one repeat unit selected from the group consisting of VPGXG, PGXGV, GXGVP, XGVPG, GVPGX, and a combination thereof, wherein V is valine, P is proline, G is glycine, and X is any amino acid except proline.

2. The SLN of claim 1, wherein the ELP is conjugated to one or more hydrophobic moieties.

3. The SLN of claim 1 or 2, wherein one or more hydrophobic moieties are conjugated to a side chain of an ELP.

4. The SLN of any one of claims 1 to 3, wherein the SLN further comprises a stabilizing agent.

5. The SLN of any one of claims 1 to 4, wherein the lipid constituting the SLN has a phase transition temperature within a range from 39°C to 60°C.

6. The SLN of any one of claims 1 to 5, wherein an average diameter of the SLN is from 10 nm to 1500 nm.

7. The SLN of any one of claims 1 to 6, wherein the SLN further comprises at least one selected from the group consisting of a biologically active agent, a pharmaceutically active agent, a magnetically active agent, an imaging agent, and a combination thereof.

8. The SLN of any one of claims 1 to 7, wherein the SLN comprises:
an ELP conjugated to one or more hydrophobic moieties;
a first lipid molecule;
a second lipid molecule; and
a stabilizing agent,
wherein the first lipid molecule is a phospholipid, the second lipid molecule is a neutral lipid molecule, and the stabilizing agent is selected from the group consisting of a sterol or its derivative, a sphingolipid or its derivative, and a combination thereof, wherein the phospholipid has an acyl group having 16 to 24 carbon atoms, and the neutral lipid comprises one or more of a monoglyceride, a diglyceride, or a triglyceride of carboxylic acids having 4 to 24 carbon atoms.

9. The SLN of claim 8, wherein the SLN comprises an ELP conjugated to one or more hydrophobic moieties, a phosphatidylcholine, triglyceride composed of a tricaprin and a trilaurin, and a cholesteryl oleate, wherein the ELP conjugated to one or more hydrophobic moiety is:
a stearoyl- or cholesteryl-V'n-NH₂, wherein n is 1 to 200, wherein V' represents one selected from the group consisting of VPGXG, PGXGV, GXGVP, XGVPG, and GVPGX, wherein V' of each location is the same or different from each other when n is 2 or greater, wherein V is valine, P is proline, G is glycine, and X is any natural or non-natural amino acid except proline, wherein X of V' of each location is same or different from each other;
a stearoyl- or cholesteryl-[V₁n₁V₂n₂]n₃-NH₂, wherein n₁, n₂, and n₃ are each independently 1 to 200, wherein V₁ and V₂ each independently represents one selected from the group consisting of VPGXG, PGXGV, GXGVP, XGVPG, and GVPGX, wherein V' of each location is the same or different from each other when n₁ and n₂ are each independently 2 or greater, wherein V is valine, P is proline, G is glycine, and X is any natural or non-natural amino acid except proline, and X of V' of each location is same or different from each other;
a stearoyl- or cholesteryl-[B(SA or Chol)n₁V₁n₂]n₃-NH₂, wherein n₁, n₂, and n₃ are each independently 1 to 200, wherein B(SA or Chol) represents one selected from the group consisting of VPGXG, PGXGV, GXGVP, XGVPG, and GVPGX, wherein V is valine, P is proline, G is glycine, and X is lysine, arginine, or histidine having an side chain amino group conjugated with a stearoyl or cholesteryl moiety, wherein V₁ represents one selected from the group consisting of VPGXG, PGXGV, GXGVP, XGVPG, and GVPGX, wherein V is valine, P is proline, G is glycine, and X is any natural or non-natural amino acid except proline, and B(SA or Chol) of each location are same or different from each other when n₁ and n₂ are each independently 2 or greater and V₁ of each location are same or different from each other when n₁ and n₂ are each independently 2 or greater; or
a stearoyl(VPGVG)n-NH₂, where n is 1 to 200, a phosphatidycholine, a triglyceride composed of a tricaprin and a trilaurin, and a cholesteryl oleate.

10. The SLN of claim 9, wherein a molar ratio of a phosphatidylcholine; a triglyceride composed of a tricaprin and a trilaurin; a cholesteryl oleate is 2 to 5: 0.1 to 3: 0 to 1, and a molar ratio of the tricaprin and the trilaurin is 1: 0.25 to 4, and the ELP conjugated to a hydrophobic moiety is 0.01 to 50wt% of phosphatidylcholine.

11. A pharmaceutical composition for delivering an active agent to a target site in a subject, the composition comprising:
pharmaceutically acceptable carriers or diluents; and
a SLN containing the active agent,
wherein the SLN is an SLN according to any one of claims 1 to 10.

12. A SLN containing an agent for use in delivering the agent to a target site in a subject, wherein the SLN is a SLN according to any one of claims 1 to 10.

13. The SLN for use of claim 12, wherein the agent is releasable from the SLN by heating the SLN.

## Patentansprüche

1. Festes Lipidnanopartikel (solid lipid nanoparticle, SLN) umfassend:
ein elastinartiges Polypeptid (ELP), das an einen hydrophoben Rest konjugiert ist; und
ein Lipidmolekül,
wobei der hydrophobe Rest eine gesättigte oder ungesättigte Kohlenwasserstoffgruppe, eine substituierte Amidgruppe mit der Formel -C(O)N(R1)(R2), wobei R1 und R2 unabhängig voneinander eine gesättigte oder ungesättigte Kohlenwasserstoffgruppe sind, oder eine gesättigte oder ungesättigte Alkoxygruppe ist, wobei das Lipidmolekül ein neutrales Lipidmolekül oder ein amphipathisches Lipidmolekül ist, und wobei das ELP wenigstens eine Wiederholungseinheit, ausgewählt aus der Gruppe bestehend aus VPGXG, PGXGV, GXGVP, XGVPG, GVPGX und einer Kombination davon, umfasst, wobei V Valin ist, P Prolin ist, G Glycin ist, und X eine beliebige Aminosäure mit Ausnahme von Prolin ist.

2. SLN nach Anspruch 1, wobei das ELP an einen oder mehrere hydrophobe Reste konjugiert ist.

3. SLN nach Anspruch 1 oder 2, wobei ein oder mehrere hydrophobe Reste an eine Seitenkette eines ELP konjugiert sind.

4. SLN nach einem der Ansprüche 1 bis 3, wobei das SLN außerdem ein Stabilisierungsmittel umfasst.

5. SLN nach einem der Ansprüche 1 bis 4, wobei das Lipid, welches Bestandteil des SLN ist, eine Phasenübergangstemperatur in einem Bereich von 39 °C bis 60°C hat.

6. SLN nach einem der Ansprüche 1 bis 5, wobei ein mittlerer Durchmesser des SLN 10 nm bis 1500 nm beträgt.

7. SLN nach einem der Ansprüche 1 bis 6, wobei das SLN außerdem wenigstens eines, ausgewählt aus der Gruppe bestehend aus einem biologisch aktiven Mittel, einem pharmazeutisch aktiven Mittel, einem magnetisch aktiven Mittel, einem bildgebenden Mittel und einer Kombination davon, umfasst.

8. SLN nach einem der Ansprüche 1 bis 7, wobei das SLN umfasst:
ein ELP, das an einen oder mehrere hydrophobe Reste konjugiert ist;
ein erstes Lipidmolekül;
ein zweites Lipidmolekül; und
ein Stabilisierungsmittel,
wobei das erste Lipidmolekül ein Phospholipid ist, das zweite Lipidmolekül ein neutrales Lipidmolekül ist, und das Stabilisierungsmittel ausgewählt ist aus der Gruppe bestehend aus einem Sterol oder seinem Derivat, einem Sphingolipid oder seinem Derivat, und einer Kombination davon, wobei das Phospholipid eine Acylgruppe mit 16 bis 24 Kohlenstoffatomen aufweist, und das neutrale Lipid eines oder mehrere von einem Monoglycerid, einem Diglycerid oder einem Triglycerid von Carbonsäuren mit 4 bis 24 Kohlenstoffatomen umfasst.

9. SLN nach Anspruch 8, wobei das SLN ein ELP, das an einen oder mehrere hydrophobe Reste konjugiert ist, ein Phosphatidylcholin, ein Triglycerid, das sich aus einem Tricaprin und einem Trilaurin zusammensetzt, und ein Cholesteryloleat umfasst, wobei das ELP, das an einen oder mehrere hydrophobe Reste konjugiert ist, folgendes ist:
ein Stearoyl- oder Cholesteryl-V'n-NH₂, wobei n 1 bis 200 ist, wobei V' eines, ausgewählt aus der Gruppe bestehend aus VPGXG, PGXGV, GXGVP, XGVPG und GVPGX, bedeutet, wobei V' von jeder Stelle gleich oder voneinander verschieden ist, wenn n 2 oder größer ist, wobei V Valin ist, P Prolin ist, G Glycin ist und X eine beliebige natürliche oder nicht natürliche Aminosäure mit Ausnahme von Prolin ist, wobei X von V' von jeder Stelle gleich oder voneinander verschieden ist;
ein Stearoyl- oder Cholesteryl-[V₁n₁V₂n₂]n₃-NH₂, wobei n₁, n₂ und n₃ jeweils unabhängig voneinander 1 bis 200 sind, wobei V₁ und V₂ jeweils unabhängig voneinander eines, ausgewählt aus der Gruppe bestehend aus VPGXG, PGXGV, GXGVP, XGVPG und GVPGX, bedeutet, wobei V' von jeder Stelle gleich oder voneinander verschieden ist, wenn n₁ und n₂ jeweils unabhängig voneinander 2 oder größer sind, wobei V Valin ist, P Prolin ist, G Glycin ist und X eine beliebige natürliche oder nicht natürliche Aminosäure mit Ausnahme von Prolin ist, und X von V' von jeder Stelle gleich oder voneinander verschieden ist;
ein Stearoyl- oder Cholesteryl-[B(SA oder Chol)n₁V₁n₂]n₃-NH₂, wobei n₁, n₂ und n₃ jeweils unabhängig voneinander 1 bis 200 sind, wobei B(SA oder Chol) eines, ausgewählt aus der Gruppe bestehend aus VPGXG, PGXGV, GXGVP, XGVPG und GVPGX, bedeutet, wobei V Valin ist, P Prolin ist, G Glycin ist und X Lysin, Arginin oder Histidin mit einer Seitenkettenaminogruppe, die mit einem Stearoyl- oder Cholesterylrest konjugiert ist, ist, wobei V₁ eines, ausgewählt aus der Gruppe bestehend aus VPGXG, PGXGV, GXGVP, XGVPG und GVPGX, bedeutet, wobei V Valin ist, P Prolin ist, G Glycin ist und X eine beliebige natürliche oder nicht natürliche Aminosäure mit Ausnahme von Prolin ist, und B(SA oder Chol) von jeder Stelle gleich oder voneinander verschieden sind, wenn n₁ und n₂ jeweils unabhängig voneinander 2 oder größer sind, und V₁ von jeder Stelle gleich oder voneinander verschieden sind, wenn n₁ und n₂ jeweils unabhängig voneinander 2 oder größer sind; oder
ein Stearoyl(VPGVG)n-NH₂, wobei n 1 bis 200 ist, ein Phosphatidylcholin, ein Triglycerid, das sich aus einem Tricaprin und einem Trilaurin zusammensetzt, und ein Cholesteryloleat.

10. SLN nach Anspruch 9, wobei ein Molverhältnis von einem Phosphatidylcholin; einem Triglycerid, das sich aus einem Tricaprin und einem Trilaurin zusammensetzt; einem Cholesteryloleat 2 bis 5: 0,1 bis 3: 0 bis 1 beträgt, und ein Molverhältnis von dem Tricaprin und dem Trilaurin 1: 0,25 bis 4 beträgt, und das ELP, das an einen hydrophoben Rest konjugiert ist, 0,01 bis 50 Gew.-% von Phosphatidylcholin beträgt.

11. Pharmazeutische Zusammensetzung zum Verabreichen eines aktiven Mittels bzw. Wirkstoffs an einem Zielort in einem Probanden, wobei die Zusammensetzung umfasst:
pharmazeutisch verträgliche Träger oder Verdünnungsmittel; und
ein SLN, das den Wirkstoff enthält,
wobei das SLN ein SLN gemäß einem der Ansprüche 1 bis 10 ist.

12. SLN, das ein Mittel enthält, zur Verwendung zum Verabreichen des Mittels an einem Zielort in einem Probanden, wobei das SLN ein SLN gemäß einem der Ansprüche 1 bis 10 ist.

13. SLN zur Verwendung nach Anspruch 12, wobei das Mittel aus dem SLN durch Erwärmen des SLN freigesetzt werden kann.

## Revendications

1. Une nanoparticule lipidique solide (SLN) comprenant:
un polypeptide de type élastine (ELP) conjugué à un fragment hydrophobe; et
une molécule lipidique,
dans laquelle le fragment hydrophobe est un groupe hydrocarbonné saturé ou insaturé, un groupe amide substitué avec la formule -C(O)N(R1)(R2) dans laquelle R1 et R2 représentent indépendamment un groupe hydrocarbonné saturé ou insaturé, ou un groupe alcoxy saturé ou insaturé, dans laquelle la molécule lipidique est une molécule lipidique neutre ou une molécule lipidique amphipathique, et dans laquelle l'ELP comprend au moins une unité de répétition sélectionnée parmi le groupe consistant en VPGXG, PGXGV, GXGVP, XGVPG, GVPGX, et une combinaison de ces derniers, dans laquelle V représente une valine, P représente une proline, G représente une glycine, et X représente n'importe quel acide aminé excepté la proline.

2. La SLN selon la revendication 1, dans laquelle l'ELP est conjugué à un ou plusieurs fragments hydrophobes.

3. La SLN selon la revendication 1 ou 2, dans laquelle un ou plusieurs fragments hydrophobes sont conjugués à une chaine latérale d'un ELP.

4. La SLN selon une quelconque des revendications 1 à 3, dans laquelle la SLN comprend en outre un agent stabilisant.

5. La SLN selon une quelconque des revendications 1 à 4, dans laquelle le lipide constituant la SLN a une température de transition de phase dans une plage de 39°C à 60°C.

6. La SLN selon une quelconque des revendications 1 à 5, dans laquelle le diamètre moyen de la SLN est de 10 nm à 1500 nm.

7. La SLN selon une quelconque des revendications 1 à 6, dans laquelle la SLN comprend en outré au moins un agent sélectionné parmi le groupe consistant en un agent biologiquement actif, un agent pharmaceutiquement actif, un agent magnétiquement actif, un agent d'imagerie, et une combinaison de ces derniers.

8. La SLN selon une quelconque des revendications 1 à 7, dans laquelle la SLN comprend:
un ELP conjugué à un ou plusieurs fragments hydrophobes;
une première molécule lipidique;
une seconde molécule lipidique; et
un agent stabilisant,
dans laquelle la première molécule lipidique est un phospholipide, la seconde molécule lipidique est une molécule lipidique neutre, et l'agent stabilisant est sélectionné parmi le groupe consistant en un stérol ou ses dérivés, un sphingolipide ou ses dérivés, et une combinaison de ces derniers, dans laquelle le phospholipide a un groupe acyle ayant 16 à 24 atomes de carbone, et le lipide neutre comprend un ou plusieurs de un monoglycéride, un diglycéride, ou un triglycéride d'acides carboxyliques ayant 4 à 24 atomes de carbone.

9. La SLN selon la revendication 8, dans laquelle la SLN comprend un ELP conjugué à un ou plusieurs fragments hydrophobes, une phosphatidylcholine, un triglycéride composé d'un tricaprine et un trilaurine et un oléate de cholestéryle, dans laquelle l'ELP conjugué à un ou plusieurs fragments hydrophobes est :
un stéaroyl- ou cholestéryl-V'n-NH₂, dans lequel n représente 1 to 200, dans lequel V' représente un élément sélectionné parmi le groupe consistant en VPGXG, PGXGV, GXGVP, XGVPG, et GVPGX, dans lequel V' de chaque emplacement sont les mêmes ou différents les uns des autres quand n représente 2 ou plus, dans lequel V représente une valine, P représente une proline, G représente une glycine, et X représente n'importe quel acide aminé naturel ou non naturel excepté la proline, dans lequel X de V' de chaque emplacement sont les mêmes ou différents les uns des autres;
un stéaroyl- ou cholestéryl -[V₁n₁V₂n₂]n₃-NH₂, dans lequel n₁, n₂, et n₃ représentent chacun indépendamment 1 à 200, dans lequel V₁ et V₂ représentent chacun indépendamment un élément sélectionné parmi le groupe consistant en VPGXG, PGXGV, GXGVP, XGVPG, et GVPGX, dans lequel V' de chaque emplacement sont les mêmes ou différents les uns des autres quand n₁ et n₂ représentent chacun indépendamment 2 ou plus, dans laquelle V représente une valine, P représente une proline, G représente une glycine, et X représente n'importe quel acide aminé naturel ou non naturel excepté la proline, et X de V' de chaque emplacement sont les mêmes ou différents les uns des autres;
un stéaroyl- ou cholestéryl-[B(SA ou Chol)n₁V,n₂]n₃-NH₂, dans lequel n₁, n₂, et n₃ représentent chacun indépendamment 1 à 200, dans lequel B(SA ou Chol) représente un élément sélectionné parmi le groupe consistant en VPGXG, PGXGV, GXGVP, XGVPG, et GVPGX, dans lequel V représente une valine, P représente une proline, G représente une glycine, et X représente une lysine, une arginine, ou une histidine ayant un groupe amino de chaine latérale conjugué à un fragment stéaroyle ou cholestéryle, dans lequel V₁ représente un élément sélectionné parmi le groupe consistant en VPGXG, PGXGV, GXGVP, XGVPG, et GVPGX, dans lequel V représente une valine, P représente une proline, G représente une glycine, et X représente n'importe quel acide aminé naturel ou non naturel excepté la proline, et B(SA ou Chol) de chaque emplacement sont les mêmes ou différents les uns des autres quand n₁ et n₂ représentent chacun indépendamment 2 ou plus et V₁ de chaque emplacement sont les mêmes ou différents les uns des autres quand n₁ et n₂ représentent chacun indépendamment 2 ou plus; ou
un stéaroyl(VPGVG)n-NH₂, où n est 1 à 200, une phosphatidylcholine, un triglycéride composé d'un tricaprine et un trilaurine et un oléate de cholestéryle.

10. La SLN selon la revendication 9, dans laquelle un rapport molaire d'une phosphatidylcholine; un triglycéride composé d'un tricaprine et un trilaurine; et un oléate de cholestéryle est de 2 à 5: 0.1 à 3: 0 à 1, et un rapport molaire du tricaprine et du trilaurine est de 1: 0.25 à 4, et l'ELP conjugué à un fragment hydrophobe est de 0.01 à 50% en poids de phosphatidylcholine.

11. Une composition pharmaceutique pour délivrer un agent actif sur un site cible chez un sujet, la composition comprenant:
des supports ou diluants pharmaceutiquement acceptable; et
une SLN contenant l'agent actif,
dans laquelle la SLN est une SLN selon une quelconque des revendications 1 à 10.

12. Une SLN contenant un agent pour utiliser en délivrant l'agent sur le site cible chez un sujet, dans laquelle la SLN est une SLN selon une quelconque des revendications 1 à 10.

13. La SLN pour utiliser selon la revendication 12, dans laquelle l'agent peut être libéré à partir de la SLN en chauffant la SLN.
